# EUROPÄISCHE PATENTANMELDUNG

(11) **EP 2 537 772 A1**
(43) Veröffentlichungstag der Anmeldung: **26.12.2012**
(21) Anmeldenummer: 11170663.6
(22) Anmeldetag: 21.06.2011
(51) Int. Cl.: B65D 47/20, A61M 39/04, F16K 15/14, B05B 11/00

(54) **Vorrichtung zur Aufnahme und Abgabe eines Fluids**

(71) Anmelder: Weibel CDS AG, 9104 Waldstatt (CH)
(72) Erfinder: Weibel. Ludwig Daniel, 9104 Waldstatt (CH)
(74) Vertreter: Hepp Wenger Ryffel AG

(57) **Zusammenfassung**

Die Erfindung betrifft eine Vorrichtung (1) zur Aufnahme oder Abgabe eines Fluids (39), umfassend ein Medikament, mit einem wenigstens bereichsweise kollabierbaren Behälter, mit einem Verschlussstück (2). Das Verschlussstück (2) umfasst einen Abgabekanal (18), welcher die Abgabeöffnung (2.1) fluidmässig mit der Entnahmeöffnung (21) verbindet, wobei ein Abgabeabschnitt (18.1) des Abgabekanals (18) mit der Abgabeöffnung (2.1) kommuniziert und ein Entnahmeabschnitt (18.2) des Abgabekanals (18) mit der Entnahmeöffnung (21) kommuniziert. Der Abgabekanal (18) umfasst eine Ventilvorrichtung (23), zwei nebeneinander auf einer Ventilfläche (6.1) angeordnete Ventilöffnungen (22,25) aufweist, von welchen eine erste Ventilöffnung (25) über den Abgabeabschnitt (18.1) des Abgabekanals (18) mit der Abgabeöffnung (2.1) kommuniziert und eine zweite Ventilöffnung (22) über den Entnahmeabschnitt (18.2) des Abgabekanals (18) mit der Entnahmeöffnung (21) kommuniziert, und eine durchgehende flexible Membran (27) vorhanden ist, welche in der Schliessstellung der Ventilvorrichtung (23) bereichsweise abhebbar an der Ventilfläche (6.1) anliegt und so die Ventilöffnungen (22,25) fluiddicht gegeneinander abschliesst.

## Beschreibung

### Technisches Gebiet

Vorrichtung zur Aufnahme oder Abgabe eines Fluids, vorzugsweise für ein Fluid umfassend ein Medikament, mit einem Behälter, insbesondere einen wenigstens bereichsweise kollabierbaren Behälter, und mit einem Verschlussstück, das Verschlussstück umfassend einen Anschlussbereich zum Anschluss des Behälters. Dabei ist im Anschlussbereich eine Entnahmeöffnung derart angeordnet, dass sie mit einem Innenraum des Behälters kommuniziert. Weiter umfasst das Verschlussstück einen Abgabebereich, in welchem zur Abgabe des Fluids eine Abgabeöffnung ausgebildet ist, sowie einen Abgabekanal, welcher die Abgabeöffnung fluidmässig mit der Entnahmeöffnung verbindet, wobei ein Abgabeabschnitt des Abgabekanals mit der Abgabeöffnung kommuniziert und ein Entnahmeabschnitt des Abgabekanals mit der Entnahmeöffnung kommuniziert. Der Abgabekanal umfasst eine Ventilvorrichtung, welche in einer Schliessstellung den Abgabekanal fluiddicht verschliesst und in einer Offenstellung einen Durchtritt des Fluids durch den Abgabekanal ermöglicht, wenn in einem Fluid im Entnahmeabschnitt ein hinreichend grosser Überdruck besteht.

### Stand der Technik

Medikamente allgemein und insbesondere parenteral, d.h. unter Umgehung des Verdauungstrakts, zu verabreichende Medikamente, werden nach ihrer Zubereitung in einen Behälter abgefüllt, der eine oder mehrere Portionen/Dosen aufnehmen kann. Derartige Behälter werden in der Regel als Primärpackung bezeichnet. Das sich in der Primärpackung befindende Medikament muss zur Abgabe an einen Patienten in der Regel in eine Abgabevorrichtung wie beispielsweise, im Falle eines zu injizierenden Medikaments, in eine Spritze transferiert werden.

Als Primärpackung sind z.B. Glas-Ampullen bekannt die mit einer speziellen Ampullensäge zu öffnen sind oder die zum Öffnen mit einer Sollbruchstelle versehen sind. Durch die so geschaffene Öffnung kann das Medikament in die Abgabevorrichtung transferiert werden. Dabei muss im Fall der Glas-Ampulle meist ein Rest in der Ampulle verbleiben, da es ansonsten beim Aufziehen der Spritze zum unerwünschten Ansaugen von Luft kommen kann. Ein späteres Wiederverschliessen der Ampulle unter Wahrung der Sterilität ist praktisch unmöglich.

Als weitere Primärpackungen sind so genannte Stechampullen (Vials oder Fiolen) bekannt. Diese weisen z.B. ein selbst schliessendes Penetrationselement für die Nadel einer Spritze auf. Zum Aufziehen der Spritze wird das Penetrationselement durchstochen und das im Behälter vorhandene Medikament in die Spritze transferiert (Aufziehen der Spritze). Der Innenraum der Stechampulle bleibt dabei weitgehend steril, womit sich derartige Primärpackungen zur mehrmaligen Flüssigkeitsentnahme eignen.

Auf derartigen Primärpackungen basierende Systeme sind aufgrund des erforderlichen Transfers in Abgabevorrichtungen allerdings aufwändig und es besteht ein erhöhtes Risiko einer Kontamination des Medikaments durch Schmutz oder Mikroorganismen. Beispielsweise in der Notfallmedizin ist das Öffnen oder Anstechen von Ampullen und der anschliessende Transfer in die Abgabevorrichtung unter Zeitdruck und gegebenenfalls in stark kontaminierten Umgebungen allerdings heikel.

Andere bekannte Systeme verzichten auf Primärpackungen und lagern die Medikamente direkt in den zur Abgabe vorgesehenen Vorrichtungen wie z.B. Spritzen. Die Abgabevorrichtungen können zur Verabreichung mit einer aufgesteckten oder eingeklebten Hohlnadel oder Kanüle versehen sein. Durch Pressen eines beweglich gelagerten Kolbens wird die Flüssigkeit aus dem Behälter durch die Kanüle injiziert. Derartige Systeme sind konstruktionsbedingt allerdings vergleichsweise teuer. Zudem müssen Vorkehrungen getroffen werden, dass die Funktionsfähigkeit der Vorrichtung und das Medikament auch eine längere Lagerdauer überstehen. Hierzu werden Kolben und Spritzenbehälter mit Beschichtungen wie z.B. Silikon versehen. Damit wird eine Substanz zusammen mit dem Medikament gelagert und verabreicht, die mit der eigentlichen Wirkung des Medikaments nichts zu tun hat und sich z.B. bei Medikamenten mit einem hohen pH-Wert nachteilig auswirken kann.

Die US 3,114,369 beschreibt einen durch Fingerdruck deformierbaren Behälter, welcher aufgrund des so erzeugbaren Überdrucks im Innenraum des Behälters eine Flüssigkeit durch eine Abgabeöffnung z.B. durch eine Hohlnadel abgibt. Die Abgabeöffnung ist durch eine penetrierbare Membran versiegelt, welche vor der Benutzung durch das hintere Ende der Injektionsnadel durchstossen werden kann. Ist die Membran jedoch einmal durchstossen, kann Flüssigkeit ungehindert austreten, was beispielsweise ein unerwünschtes Nachtröpfeln zur Folge haben kann.

Die DE 32 24 656 beschreibt ebenfalls einen deformierbaren Behälter zur Aufbewahrung von medizinischen Flüssigkeiten, welcher durch Drücken durch eine Abgabeöffnung zu entleeren ist. Dabei ist eine Ventilvorrichtung an einem Verschlussstück des Behälters vorgesehen, welche eine einmal durch die Ventilvorrichtung ausgetretene Flüssigkeit am Zurückfliessen in den Behälter hindert. Die Ventilvorrichtung stellt zudem sicher, dass die im Behälter enthaltene medizinische Flüssigkeit nicht ungewollt auslaufen kann. Allerdings weist das Verschlussstück im Ventilbereich konstruktionsbedingt Taschen und Toträume auf, welche zu Lufteinschlüssen führen können. Eine direkte Injektion des Fluids aus dem Behälter in den Körper eines Lebewesens ist daher in der Regel nicht möglich. Ausserdem kann aufgrund der Toträume mit Schmutz oder Mikroorganismen kontaminierte Flüssigkeit auf unbestimmte Zeit im Verschlussstück verweilen, womit eine sterile Umgebung kaum gewährleistet werden kann.

Es ist daher die Aufgabe der Erfindung, die Nachteile des Standes der Technik zu überwinden, insbesondere eine Vorrichtung zur Aufnahme und zur Abgabe eines Fluids zu schaffen, insbesondere für ein Fluid umfassend ein Medikament, welche einfach in der Konstruktion und Herstellung sowie einfach und sicher in der Handhabung ist. Weiter besteht die Aufgabe der Erfindung darin, eine Vorrichtung zur Aufnahme und Abgabe einer Flüssigkeit zu schaffen, welche eine mehrmalige Fluidabgabe erlaubt, ohne dass das Fluid ungewollt auslaufen oder durch Schmutz oder Mikroorganismen kontaminiert werden kann. Weiter besteht die Aufgabe der Erfindung darin, eine Vorrichtung zur Aufnahme und Abgabe eines Fluids bereitzustellen, welche für eine direkte parenterale Verabreichung des Fluids aus dem Behälter geeignet ist.

### Darstellung der Erfindung

Die Lösung der Aufgabe ist durch die Merkmale des Anspruchs 1 definiert. Gemäss der Erfindung umfasst die Vorrichtung zur Aufnahme oder Abgabe eines Fluid, vorzugsweise für ein Fluid umfassend ein Medikament, einen Behälter, insbesondere einen wenigstens bereichsweise kollabierbaren Behälter, und ein Verschlussstück. Das Verschlussstück umfasst einen Anschlussbereich zum Anschluss des Behälters, wobei im Anschlussbereich eine Entnahmeöffnung derart angeordnet ist, dass sie mit einem Innenraum des Behälters kommuniziert. Weiter umfasst das Verschlussstück einen Abgabebereich, in welchem zur Abgabe des Fluids eine Abgabeöffnung ausgebildet ist, sowie einen Abgabekanal, welcher die Abgabeöffnung fluidmässig mit der Entnahmeöffnung verbindet. Dabei kommunizieren ein Abgabeabschnitt des Abgabekanals mit der Abgabeöffnung und ein Entnahmeabschnitt des Abgabekanals mit der Entnahmeöffnung. Der Abgabekanal umfasst eine Ventilvorrichtung, welche in einer Schliessstellung den Abgabekanal fluiddicht verschliesst und in einer Offenstellung einen Durchtritt des Fluids durch den Abgabekanal ermöglicht, wenn in einem Fluid im Entnahmeabschnitt ein hinreichend grosser Überdruck besteht. Die Erfindung zeichnet sich dadurch aus, dass die Ventilvorrichtung zwei nebeneinander auf einer Ventilfläche, welche insbesondere auf einer gemeinsamen Ventilfläche angeordnet sind, angeordnete Ventilöffnungen aufweist, von welchen eine erste Ventilöffnung über den Abgabeabschnitt des Abgabekanals mit der Abgabeöffnung kommuniziert und eine zweite Ventilöffnung über den Entnahmeabschnitt des Abgabekanals mit der Entnahmeöffnung kommuniziert, und eine durchgehende flexible Membran vorhanden ist, welche in der Schliessstellung der Ventilvorrichtung bereichsweise abhebbar an der Ventilfläche anliegt und so die Ventilöffnungen fluiddicht gegeneinander abschliesst.

Es versteht sich, dass die Ventilfläche sich aus mehreren Abschnitten zusammensetzen kann, welche beispielsweise unter einem Winkel aneinander stossen oder gegeneinander abgestuft sind. Bevorzugt sind jedoch die beiden Ventilöffnungen auf einer gemeinsamen Ventilfläche des Verschlussstücks angeordnet. Damit kann die flexible Membran auf einfache Weise auf der gemeinsamen Ventilfläche angeordnet sein und so die Ventilöffnungen fluiddicht gegeneinander abschliessen.

Übersteigt ein Druck im Fluid im Innenraum des Behälters einen gewissen Schwellenwert, kann über das Fluid im Entnahmeabschnitt die flexible Membran von der Ventilfläche im Bereich zwischen den Ventilöffnungen abgehoben werden, wodurch zwischen Ventilfläche und Membran eine Passage für das Fluid geschaffen wird. Das Fluid kann so zur benachbart angeordneten, abgabeseitigen Ventilöffnung und damit in den Abgabeabschnitt des Abgabekanals gelangen. Mit anderen Worten ist die flexible Membran derart abhebbar auf der Ventilfläche angeordnet, dass in Abhängigkeit eines Überdrucks in einem Fluid im Entnahmeabschnitt des Abgabekanals die Membran abgehoben werden kann und so die Ventilvorrichtung in eine Offenstellung gebracht werden kann. Die flexible Membran ermöglicht somit in der Offenstellung der Ventilvorrichtung einen Fluidschluss zwischen den beiden Abschnitten des Abgabekanals. Membran und Ventilfläche begrenzen dabei einen den Fluidschluss herstellenden Fluidkanal. Aufgrund des erforderlichen Überdrucks ist sichergestellt, dass auch in der Offenstellung der Ventilvorrichtung kein Rückfluss von allfälligen Fluidresten aus dem abgabeseitigen Abschnitt in den entnahmeseitigen Abschnitt erfolgt.

Die flexible Membran liegt insbesondere in einem Bereich zwischen den Ventilöffnungen fluiddicht und abhebbar an der Ventilfläche an. In weiteren Bereichen der Ventilfläche kann die Membran mit Vorteil fest mit der Ventilfläche verbunden, d.h. z.B. nicht abhebbar befestigt sein. Damit kann in der Offenstellung der Ventilvorrichtung zwischen den Ventilöffnungen ein die Ventilöffnungen fluidmässig verbindender Fluidkanal definiert werden. Mit Vorteil kann die flexible Membran dabei auch elastisch ausgebildet sein, sodass die Membran bei Nachlassen des Überdrucks von selbst wieder auf die Ventilfläche abgesenkt werden kann und/oder in abgesenktem Zustand zur besseren Fluidabdichtung eine Andruckkraft auf die Ventilfläche ausüben kann. Zur einfachen Erzeugung des Überdrucks im Fluid ist der Behälter mit Vorteil wenigstens bereichsweise kollabierbar ausgebildet (siehe unten). In anderen Ausführungsformen kann der Behälter z.B. als Balg aus einem elastischen Material gefertigt sein oder aber auch erforderlichenfalls feste Wände aufweisen, d.h. nicht kollabierbar ausgebildet sein. Ein Überdruck in einem Fluid im Behälter kann in diesem Fall z.B. mit einem Kolben nach Art einer Spritze erzeugt werden. Es versteht sich, dass diese Ausführungsformen von kollabierbaren Behältern nur exemplarischen Charakter haben und es wird auf den reichhaltigen einschlägigen Stand der Technik für weitere mögliche Ausführungsformen verwiesen.

Die Ventilvorrichtung des Verschlussstücks ist erfindungsgemäss derart ausgebildet, dass der Entnahmeabschnitt und der Abgabeabschnitt in einem Ruhezustand der Ventilvorrichtung, d.h. wenn die Membran im abhebbaren Bereich an der Ventilfläche anliegt, durch die flexible Membran gegeneinander abgetrennt sind. Bevorzugt liegt die flexible Membran hierzu wenigstens in den Randbereichen der Ventilöffnungen fluiddicht an der Ventilfläche an.

Konstruktionsbedingt weist die Ventilvorrichtung eine bidirektionale Funktionalität auf. Bei hinreichend grossem Überdruck in einem Fluid im Abgabeabschnitt des Abgabekanals kann analog zum oben beschriebenen Durchtritt vom Entnahmeabschnitt zum Abgabeabschnitt auch ein Fluiddurchtritt in umgekehrter Richtung erfolgen. Dies hat den Vorteil, dass der Behälter grundsätzlich über die Abgabeöffnung durch den Abgabekanal befüllt werden kann. Denkbar sind beispielsweise teilweise evakuierte Beutel oder Tuben, welche mit dem Verschlussstück versehen sind und auf diese Weise befüllt werden können. Die Vorrichtung bzw. der Behälter oder das Verschlussstück können auch eine Entlüftungsvorrichtung, z.B. ein Einwegventil, umfassen, sodass beim Befüllen allfällige Luft aus dem Innenraum des Behälters entweichen kann.

Ein Schwellenwert des Überdrucks, welcher zur Herstellung des Fluidschlusses zwischen den Ventilöffnungen erforderlich ist, ist mit Vorteil derart gewählt, dass ein versehentlicher Durchtritt des Fluids zwischen den beiden Abschnitten weitgehend ausgeschlossen ist. Sollte es dennoch zu einem ungewollten Durchtritt kommen, indem der Behälter z.B. unbeabsichtigt gequetscht wird, erfolgt ein Durchtritt des Fluids vom Behälter nach aussen, wobei keine Kontamination des Behälterinhalts erfolgt. Durch Wahl der Flexibilität der Membran können unterschiedliche Schwellenwerte eingestellt werden, womit die Ventilvorrichtung den Erfordernissen der jeweiligen Anwendung angepasst werden kann (z.B. an eine Viskosität des Fluids).

Das Fluid, welches im Behälter vorhanden sein kann, umfasst vorzugsweise ein Medikament, insbesondere ein parenteral, oral oder topisch anzuwendendes Medikament. Die Vorteile der erfindungsgemässen Konstruktion erschliessen sich allerdings auch unmittelbar in anderen Medizinalbereichen. Als beispielhafte und nicht erschöpfende Aufzählung ist der Behälter z.B. auch zur Anwendungen bei Desinfektionsmitteln sowie Reinigungsmitteln oder Spülungen geeignet. Denkbare Anwendungen der Vorrichtung zur Aufnahme und Abgabe eines Fluids sind aber nicht auf den Medizinalbereich beschränkt. Die erfindungsgemässe Vorrichtung ist beispielsweise auch bei kosmetischen Fluiden oder im Lebensmittelbereich, z.B. für viskose Lebensmittel wie Saucen (Ketchup, Senf etc.) besonders geeignet. In der Regel ist in diesen Bereichen zwar keine vollständige Wahrung der Sterilität des Fluids im Behälter erforderlich. In Anbetracht der Anwendung derartiger Fluide am menschlichen Körper bzw. durch deren Einnahme ist jedoch auch in diesem Fall auf besondere Hygiene zu achten, weshalb die erfindungsgemässe Vorrichtung auch bei diesen Anwendungen vorteilhaft sein kann. Allgemein ist die erfindungsgemässe Vorrichtung für alle Anwendungsbereiche geeignet, in welchen erhöhte Anforderungen zur Vermeidung einer Kontamination mit Schmutz oder Mikroorganismen eines Fluids in der Vorrichtung bestehen und eine einfach zu kontrollierende und dosierende Fluidabgabe gewünscht ist.

Besonders bevorzugt ist der Behälter der erfindungsgemässen Vorrichtung, zumindest teilweise, kollabierbar ausgebildet, vorzugsweise als Beutel oder Tube. Eine Behälterwand des Behälters ist dabei in der Regel wenigstens bereichsweise flexibel ausgebildet, sodass im Behälter durch zusammendrücken der Behälterwand ein Überdruck aufgebaut werden kann. Mit anderen Worten ist wenigstens ein Bereich des Behälters derart ausgebildet und beschaffen, dass wenigstens in diesem Bereich durch Deformation der Behälterwand ein Überdruck im Innenraum des Behälters aufgebaut werden kann, sodass ein im Innenraum des Behälters vorhandenes Fluid durch die Entnahmeöffnung in den Abgabekanal des Verschlussstücks pressbar und über die Ventilvorrichtung der Abgabeöffnung zuführbar ist.

Auf diese Weise kann z.B. durch Quetschen mit den Fingern oder über eine Abgabevorrichtung der erforderliche Überdruck im Fluid erzeugt werden, um die Ventilvorrichtung von der Schliessstellung in die Offenstellung zu bringen. Insbesondere kann der Behälter z.B. aus einem Folienmaterial gefertigte Behälterwände aufweisen, welche z.B. in zwei Lagen aufeinander liegen und in den Randbereichen über eine Verbindungsnaht miteinander verbunden sind (beutelartiger Behälter). Die zwei Folienlagen können dabei von getrennten Folienlagen bereitgestellt werden oder von einer einzelnen, gefalteten Folienlage. Die Verbindungsnaht kann beispielsweise eine Klebung oder eine Schweissung umfassen. Der Behälter kann aber auch eine zu einer Röhre gerollte und mit sich selbst verbundene Folie aufweisen, welche an einem Längsende der Röhre flachgedrückt und z.B. verschweisst ist (tubenartiger Behälter).

Bevorzugt umfasst die Behälterwand einen Kunststoff, insbesondere einen beschichteten Kunststoff, vorzugsweise ein Laminat. Eine innere Schicht, d.h. eine Schicht, welche den Innenraum begrenzt, kann z.B. besonders verträglich mit einem spezifischen Fluid ausgebildet sein, während eine äussere Schicht z.B. die mechanischen Eigenschaften des Behälters bestimmt.

Das Verschlussstück kann im Anschlussbereich ein Kopplungsmittel wie beispielsweise ein Schraubgewinde oder ein Bajonett zum Anschluss des Behälters aufweisen, z.B. nach Art einer Zahnpastatube, auf welche anstatt eines Deckels das Verschlussstück mit Ventilvorrichtung aufgeschraubt ist.

In einer besonders bevorzugten Ausführungsform weist das Verschlussstück im Anschlussbereich jedoch wenigstens eine Anschlussfläche auf, an welcher ein Anschlussabschnitt der Behälterwand direkt befestigbar ist. Verschlussstück und Behälterwand bilden somit eine fest verbundene Einheit, wenn die Behälterwand an der Anschlussfläche befestigt ist. Bevorzugt ist die Behälterwand mit der Anschlussfläche verschweisst oder verklebt, insbesondere beispielsweise ultraschall- oder laserverschweisst. Damit ergibt sich bei einfacher Herstellung eine sichere Verbindung, welche eine fluiddichte und nicht lösbare Verbindung des Behälters mit dem Verschlussstück sicherstellt.

Mit Vorteil ist der Anschlussabschnitt der Behälterwand flexibel ausgebildet und die Ventilfläche mit Ventilöffnungen ist auf der wenigstens einen Anschlussfläche des Verschlussstücks derart angeordnet, dass ein Bereich des Anschlussabschnitts der Behälterwand die flexible Membran der Ventilvorrichtung bildet, insbesondere die Ventilöffnungen überdeckt. Die Behälterwand kann auf diese Weise eine Zusatzfunktion als Teil der Ventilvorrichtung erfüllen, was eine kostengünstige und einfache Konstruktion der erfindungsgemässen Vorrichtung ermöglicht. In Varianten kann die flexible Membran auch als separates Teil unabhängig von einer Behälterwand ausgebildet sein. Denkbar ist beispielsweise eine flexible Manschette, welche über die z.B. auf einer Mantelfläche eines zylindrischen Abschnitts ausgebildete Ventilfläche gestülpt ist.

Um die Konstruktion der Vorrichtung weiter zu vereinfachen, weist das Verschlussstück in einer bevorzugten Ausführungsform im Anschlussbereich einen im Wesentlichen zylindrisch ausgebildeten Abschnitt auf, wobei die wenigstens eine Anschlussfläche auf einer äusseren Mantelfläche des zylindrischen Abschnitts angeordnet ist.

Durch den zylindrischen Abschnitt kann das Verschlussstück auf einfache Weise in eine endseitige Öffnung eines z.B. tubenförmig ausgebildeten Behälters eingesetzt und dort befestigt werden. In Varianten kann der Abschnitt auch konisch ausgebildet sein, entsprechend einer beispielsweise divergierenden Form des Behälters. Bevorzugt weist das Verschlussstück jedoch einen zylindrisch ausgebildeten Abschnitt auf, welcher bei einer Vielzahl von Formen der Behälterwand wie beispielsweise Tuben und Beuteln angewendet werden kann. Der zylindrische Abschnitt kann je nach Erfordernis und/oder Behälterform beispielsweise eine kreisförmige Grundfläche aufweisen, aber auch einen eckigen oder elliptischen Querschnitt haben.

In einer bevorzugten Ausführungsform umfasst die Mantelfläche des zylindrischen Abschnitts neben der wenigstens einen Anschlussfläche eine weitere Anschlussfläche, wobei die beiden Anschlussflächen paarweise in einer gemeinsamen, insbesondere zur Zylinderachse parallel angeordneten, Kante zusammenlaufen, sodass der zylindrische Abschnitt eine weitgehend linsenförmige Grundfläche aufweist.

Hierzu sind die beiden Abschlussflächen bevorzugt gekrümmt ausgebildet und laufen in der jeweils gemeinsamen Kante derart zusammen, dass die Behälterwände, welche auf den Anschlussflächen befestigt sind, an den Kanten lückenlos aufeinander zu liegen kommen. Linsenförmig versteht sich dabei in Analogie zum Querschnitt einer bikonvexen optischen Linse. Die Anschlussflächen bilden dabei die Mantelfläche des Verschlussstücks und laufen in einer Richtung quer zur Zylinderachse in jeweils einer gemeinsamen Kante zusammen. Dabei sind die Anschlussflächen, bevorzugt kantenlos, gekrümmt, wodurch sich die Behälterwände glatt und ohne Knicke daran befestigen lassen. Es versteht sich, dass die Linsenform des Querschnitts abgeflacht oder auf andere Weise modifiziert sein kann.

Das Verschlussstück lässt sich somit auf einfache Weise mit einem als Beutel oder Tube ausgebildeten Behälter verbinden, wobei das Anschlussstück in eine Verbindungsnaht der Behälterwand bzw. der Behälterwände eingesetzt ist. Die Anschlussabschnitte der Behälterwände umgreifen dabei das Verschlussstück an den Anschlussflächen und können an den Kanten der Mantelfläche direkt aufeinander angeordnet sein. Mit Vorteil ist die Entnahmeöffnung an der Grundfläche des Verschlussstücks ausgebildet, welche im Innenraum des Behälters angeordnet ist und diesen begrenzt. Der Abgabebereich ist der Grundfläche bevorzugt in Richtung der Zylinderachse gegenüberliegend angeordnet und umfasst mit Vorteil eine weitgehend parallel zur Grundfläche angeordnete Stirnseite.

Die erfindungsgemässe Vorrichtung kann zwar grundsätzlich wie oben beschrieben über den Abgabekanal befüllt werden. Mit Vorteil weist das Verschlussstück jedoch einen zusätzlichen Füllkanal zum Befüllen des Behälters auf, welcher eine Anschlussöffnung zum Anschliessen einer Fluidquelle im Abgabebereich und eine Füllöffnung im Anschlussbereich fluidmässig verbindet, wobei die Füllöffnung mit dem Innenraum des Behälters kommuniziert.

Der zusätzliche Füllkanal mit zugehöriger Anschlussöffnung und Füllöffnung vereinfacht das Befüllen des Behälters über das Verschlussstück. Über den Füllkanal kann das Fluid in den Behälter eingebracht werden, wobei der Abgabekanal als Entlüftungskanal zum Entweichen allfälliger im Behälterinnenraum vorhandener Luft funktionieren kann. Der Behälter kann somit ohne Lufteinschlüsse vollständig mit dem Fluid gefüllt werden und ist somit insbesondere auch für parenteral zu verabreichende medizinische Fluide geeignet.

Das Befüllen des Behälters erfolgt in der Regel unter kontrollierten Bedingungen, d.h. gegebenenfalls unter sterilen Bedingungen. Es ist daher nicht erforderlich, dass der zur einmaligen Verwendung beim Befüllen vorgesehene Füllkanal besondere Vorkehrungen zum Schutz des Behälterinnenraums vor einer ungewünschten Kontamination umfassen muss. Der Füllkanal kann daher insbesondere auf einfache Weise ohne Ventilvorrichtung ausgebildet sein. Es versteht sich allerdings, dass auch im Füllkanal eine Ventilvorrichtung analog der Ventilvorrichtung des Abgabekanals ausgebildet sein kann, sofern dies gewünscht sein sollte.

Indem die Anschlussöffnung im Abgabebereich angeordnet ist, kann eine Fluidquelle auf einfache Weise von aussen her angeschlossen werden. Insbesondere kann die Anschlussöffnung nahe der Abgabeöffnung auf einer Stirnseite des Verschlussstücks ausgebildet sein, sodass Synergien hinsichtlich beispielsweise Anschlusskappen oder Kopplungsmitteln im Anschlussbereich für beide Öffnungen benutzt werden können (siehe unten).

Besonders vorteilhaft ist eine Ausführungsform der Vorrichtung, bei welcher das Verschlussstück im Abgabebereich ein Kopplungselement umfasst, welches zum direkten oder indirekten Ankoppeln der Vorrichtung an eine zusätzliche Komponente oder ein fluidführendes System ausgebildet ist. Bevorzugt umfasst das Kopplungselement dabei einen Fluidkanal, welcher an die Abgabeöffnung anschliesst und an einer Abgabeöffnung des Kopplungsteils mündet.

Bevorzugt ist das Kopplungselement an einer Stirnseite des Verschlussstücks ausgebildet, sodass die Vorrichtung auf einfache Weise angeschlossen werden kann. Das Kopplungselement kann beispielsweise eine Schraub- oder Steckverbindung oder ein Bajonett aufweisen. Bevorzugt umfasst das Kopplungselement jedoch einen vom Verschlussstück weg sich verjüngenden Kopplungskonus, welcher durch konischen Formschluss auf bekannte Weise eine fluiddichte und sichere Ankopplung ermöglicht. Mit Vorteil verläuft der Fluidkanal in Längsrichtung des Kopplungskonus. Der Kopplungskonus kann insbesondere als bekannte Luer-Lock Verbindung ausgebildet sein, vorzugsweise als Aussenkonus eines Luer-Locks. Mit Vorteil ist das Kopplungsmittel des Verschlussstücks direkt am Verschlussstück angeformt, insbesondere einstückig mit dem Verschlussstück ausgebildet.

Es versteht sich, dass kein Kopplungselement vorhanden zu sein braucht und für gewisse Anwendungen mit Vorteil z.B. eine Hohlnadel oder Spülnadel direkt am Verschlussstück befestigt, z.B. in die Abgabeöffnung eingesetzt, sein kann.

Insbesondere im Bereich von Dialysesystemen ist eine weitere Ausführungsform eines Kopplungselements bevorzugt, welches ein rohrförmiges Gehäuse umfasst, wie z.B. einen Rohrabschnitt, mit einem Lumen zur Führung eines Fluids, wie z.B. des Körperfluids eines Dialysepatienten. Das Gehäuse weist wenigstens eine Zugangsöffnung auf, welche in der Gehäusewand ausgebildet ist und mit der Abgabeöffnung des Verschlussstücks der Vorrichtung kommuniziert. Durch die Zugangsöffnung kann das Fluid aus der Vorrichtung in das Lumen eingebracht werden. Weiter ist mit Vorteil eine Ventilvorrichtung im Kopplungselement vorhanden, welche einen Durchtritt von Flüssigkeit durch die Zugangsöffnung in das Lumen zulässt und in Gegenrichtung sperrt.

Mit Vorteil weist das Kopplungselement in diesem Fall wenigstens einen zusätzlichen Injektionsport auf, über welchen mit einer externen Vorrichtung eine Medikation in das Lumen eingebracht werden kann. "Injektionsport" bezeichnet hier und im Folgenden eine verschliessbare oder selbstschliessende Zugangsöffnung zur fluiddichten Ankopplung einer medizinischen Einrichtung zur Abgabe einer Medikation an ein im Lumen geführtes Fluid. Ebenso können je nach Ausbildung des Injektionsports im Bedarfsfall auch Proben des im Lumen geführten Fluids entnommen werden.

Derartige Injektionsports können mit einem hygienisch unbedenklichen und einfach zu reinigenden Anschluss für die medizinische Einrichtung versehen sein, wie beispielsweise einem so genannten "abtupfbaren Ventil" ("swabable valve") gemäss der US 6,651,956 (Halkey-Roberts Corporation). Injektionsports im vorliegenden Verständnis können aber auch ein penetrierbares und selbst dichtendes Septum umfassen, sodass eine Medikation auch mit einer Injektionsnadel eingebracht werden kann. Das Septum überdeckt dabei bevorzugt eine Öffnung in der Gehäusewand und ist beispielsweise durch ein Ultraschallverfahren mit dem Gehäuse verschweisst. Der Querschnitt des Lumens wird somit durch das Spetum nicht oder nur geringfügig verändert, womit das Fluid im Lumen weitgehend unbeeinflusst strömen kann.

Mit Vorteil schliesst das Verschlussstück der Vorrichtung, bevorzugt mit der Stirnseite im Abgabebereich, aussenseitig seitlich am rohrförmigen Gehäuse an, sodass beide Enden des rohrförmigen Gehäuses frei zum Anschluss an ein fluidführendes System sind. Verschlussstück und Gehäuse können dabei als einstückiges Formteil ausgebildet sein. Endseitig kann das rohrförmige Gehäuse Kupplungsvorrichtungen zum Anschluss an einem oder beiden Enden an das fluidführende System aufweisen. In Varianten kann das rohrförmige Gehäuse einseitig abgeschlossen oder direkt als Abschnitt eines Schlauchs oder Rohrs ausgebildet sein.

In einer bevorzugten Ausführungsform umfasst die Vorrichtung eine zusätzliche Anschlusskappe, welche im Anschlussbereich auf das Verschlussstück aufsetzbar ist. Bevorzugt ist die Anschlusskappe derart ausgebildet, dass sie auf ein allenfalls vorhandenes Kopplungselement des Verschlussstücks aufsetzbar und an dieses koppelbar ist.

Die Anschlusskappe verschliesst mit Vorteil in aufgesetztem Zustand die Anschlussöffnung im Abgabebereich des Verschlussstücks. Hierzu kann die Anschlusskappe wenigstens einen Verschlusszapfen aufweisen, welcher derart angeordnet und bemessen ist, dass er bei aufgesetzter Anschlusskappe in die Anschlussöffnung am Verschlussstück einbringbar ist und diese fluiddicht verschliesst. Die Anschlusskappe kann in diesem Fall nach dem Befüllen des Behälters aufgesetzt werden, womit die Anschlussöffnung versiegelt ist, sodass kein Fluid austreten kann oder allfällige Verschmutzungen in den Füllkanal eintreten können. Bei mehreren möglichen Ausrichtungen zum Aufsetzten der Anschlusskappe auf das Verschlussstück können mehrere Verschlusszapfen derart ausgebildet sein, dass in jeder möglichen Ausrichtung der Anschlusskappe einer der Verschlusszapfen in der Anschlussöffnung angeordnet ist.

Mit Vorteil umfassen die Anschlusskappe und das Verschlussstück zur Verrastung der Anschlusskappe in aufgesetztem Zustand am Verschlussstück miteinander in Eingriff bringbare Rastmittel, welche verhindern, dass die Anschlusskappe ungewollt vom Verschlussstück abgenommen werden kann.

Bevorzugt umfasst die Anschlusskappe ein Kopplungsmittel zur fluidkommunizierenden Ankopplung der Abgabeöffnung an ein fluidführendes System, insbesondere einen Aussenkonus eines Luer-Locks. Das Kopplungsmittel weist dabei bevorzugt einen Fluidkanal auf, sodass in aufgesetztem Zustand der Anschlusskappe der Fluidkanal mit der Abgabeöffnung des Verschlussstücks kommuniziert.

Die Anschlusskappe kann aber auch direkt mit einer Hohlnadel, Spülnadel oder anderen Vorrichtung zur Abgabe oder Verabreichung des Fluids versehen sein, welche bei aufgesetzter Anschlusskappe mit der Abgabeöffnung des Behälters kommuniziert und beispielsweise eine direkte parenterale Verabreichung des Fluids durch Injektion oder Infusion ermöglicht.

Das Kopplungsmittel der Anschlusskappe kann gemäss einem normierten Standard ausgebildet sein. In diesem Fall kann der Behälter unabhängig von einer, allenfalls nicht normierten, Ausbildung des Kopplungselements des Verschlussstücks über die Anschlusskappe an einen normierten Anschluss eines fluidführenden Systems angekoppelt werden. Die Anschlusskappe funktioniert in diesem Fall als Anschlussadapter.

Es versteht sich, dass das Kopplungsmittel der Anschlusskappe an spezifische Bedürfnisse angepasst sein kann. Eine insbesondere im Bereich von Dialysesystemen bevorzuge Ausführungsform eines Kopplungsmittels der Anschlusskappe ist deshalb analog dem oben beschriebenen Kopplungsmittel des Verschlussstücks mit rohrförmigem Gehäuse ausgebildet. In diesem Fall schliesst allerdings die Anschlusskappe (und nicht das Verschlussstück selbst) seitlich an das rohrförmige Gehäuse an und ist z.B. angeformt. Die Anschlusskappe zusammen mit dem rohrförmigen Gehäuse kann als einstückiger Formkörper ausgebildet sein. Die Anschlusskappe ist dabei derart ausgestaltet, dass bei auf das Verschlussstück aufgesetzter Anschlusskappe die Abgabeöffnung des Verschlussstücks mit der Zugangsöffnung im rohrförmigen Gehäuse kommuniziert.

Es versteht sich, dass die Anschlusskappe auch ohne Kopplungsmittel ausgebildet sein kann und z.B. Applikationsmittel für das Fluid aufweist. Insbesondere kann beispielsweise ein Schwamm an der Anschlusskappe vorgesehen sein, welcher über einen mit der Abgabeöffnung des Verschlussstücks kommunizierenden Fluidkanal der Anschlusskappe direkt mit dem Fluid aus dem Behälter versorgt werden kann. Eine derartige Ausführungsform ist beispielsweise für eine Fluid zur Desinfektion besonders vorteilhaft, da das Fluid direkt aus der erfindungsgemässen Vorrichtung an den Schwamm zur Applikation abgegeben werden kann. Dabei kann das Applikationsmittel beispielsweise mehrere Lagen von Schutzhüllen aus z.B. Netz und/oder Mikrofilter aufweisen, welche über den Schwamm gezogen sind. Insbesondere können die einzelnen Lagen abnehmbar ausgebildet sein, sodass nach einer Grobreinigung eine erste Lage abgenommen werden kann um mit einer zweiten, noch sauberen Lage eine Feinreinigung durchzuführen. Zur endgültigen Desinfektion kann auch die zweite Lage abgenommen werden, sodass die endgültige Desinfektion mit dem noch sauberen Schwamm durchgeführt werden kann. Es versteht sich, dass je nach Anforderungen auch weitere oder weniger Lagen geeigneter Materialien vorhanden sein können. Ebenso versteht es sich, dass ein mehrlagig umhüllter Schwamm zur Applikation eines Desinfektionsmittels in mehreren Schritten auch losgelöst von der erfindungsgemässen Vorrichtung zur Aufnahme und Abgabe eines Fluids bei anderen Vorrichtungen zur Abgabe eines Fluids mit Vorteil angewendet werden kann.

In anderen Ausführungsformen kann die Anschlusskappe auch einzig zum fluiddichten Verschluss der Anschlussöffnung und/oder der Abgabeöffnung des Verschlussstücks ausgebildet sein.

Bevorzugt weist die Anschlusskappe einen weitgehend komplementär zum Kopplungselement des Verschlussstücks ausgebildeten Aufnahmeraum auf, in welchem das Kopplungselement bei aufgesetzter Anschlusskappe aufnehmbar ist.

Bevorzugt ist der Aufnahmeraum in dem mit Vorteil als Luer-Aussenkonus ausgebildeten Kopplungsmittel der Anschlusskappe ausgebildet. Damit lässt sich ein beispielsweise als Kopplungskonus ausgebildetes Kopplungselement des Verschlussstücks des Behälters Platz sparend, ähnlich gestapelten Bechern, in der Anschlusskappe bzw. in deren Kopplungsmittel unterbringen.

Bevorzugt weist die Anschlusskappe ein Verschlussmittel auf, welches derart ausgebildet ist, dass bei aufgesetzter Anschlusskappe die Ventilvorrichtung vom Verschlussmittel in der Schliessstellung fixierbar ist.

Das Verschlussmittel ermöglicht somit eine Blockierung der Ventilvorrichtung in der Schliessstellung, womit der Behälter z.B. für Lagerzwecke fest verschlossen ist, sodass auch bei einem entsprechenden Überdruck im Innenraum des Behälters kein Fluid austreten kann. Mit anderen Worten ist die Ventilvorrichtung derart in der Schliessstellung fixiert, dass der Abgabekanal unterbrochen ist, sodass das Fluid oder Schmutz durch den Abgabekanal weder in den Behälter eintreten noch aus diesem austreten kann.

Mit Vorteil ist das Verschlussmittel dabei derart ausgebildet und angeordnet, dass die flexible Membran im Bereich zwischen den Ventilöffnungen durch das Verschlussmittel fest an die Ventilfläche pressbar ist.

Das Verschlussmittel kann dabei, insbesondere federnd, beweglich an der Anschlusskappe ausgebildet sein. Bevorzugt ist das Verschlussmittel vor dem Aufsetzen der Anschlusskappe auf das Verschlussstück in einer Bereitschaftsstellung. Nach dem Aufsetzen der Anschlusskappe kann das Verschlussmittel z.B. von einem Benutzer oder einer Produktionsmaschine in eine Aktivstellung gebracht werden, in welcher es mit der flexiblen Membran zusammenwirkt. In der Aktivstellung kann das Verschlussmittel bevorzugt in einer Schliessstellung derart blockiert werden, dass die Membran vom Verschlussmittel fluiddicht an die Ventilfläche gepresst ist. Die Blockierung des Verschlussmittels kann beispielsweise über eine Verschlusskappe einer Abgabevorrichtung erfolgen, in welche die erfindungsgemässe Vorrichtung einsetzbar bzw. eingesetzt ist. In aufgesetztem Zustand wirkt die Verschlusskappe in diesem Fall derart mit dem Verschlussmittel zusammen, blockiert z.B. das Verschlussmittel, dass dieses die Membran zwischen Ventilfläche und Verschlussmittel festklemmt. Zur Benutzung der Abgabevorrichtung wird in diesem Fall die Abschlusskappe abgenommen, womit das Verschlussmittel zwar in der Aktivstellung verbleibt, aber eine Freigabestellung einnimmt. In der Freigabestellung kann das Verschlussmittel zwar weiterhin mit der Membran zusammenwirken, die Membran ist allerdings von der Ventilfläche abhebbar, sodass bei Betätigung der Abgabevorrichtung das Fluid aus der Vorrichtung abgegeben werden kann. Das Verschlussmittel kann die Membran beispielsweise weiterhin gegen die Ventilfläche drücken, erlaubt jedoch ein Abheben der Membran von der Ventilfläche. Hierzu kann das Verschlussmittel beispielsweise federnd an der Anschlusskappe gelagert sein. Je nach Ausbildung des Verschlussmittels oder der Lagerung an der Anschlusskappe kann somit weitgehend unabhängig von den Materialeigenschaften der flexiblen Membran durch den Auflagedruck in der Freigabestellung ein Schwellenwert für den erforderlichen Überdruck zum Übergang in die Offenstellung der Ventilvorrichtung vorgegeben werden.

Das Verschlussmittel kann grundsätzlich aber auch fest und unbeweglich an der Anschlusskappe angebracht sein und bereits aufgrund des Aufsetzens der Anschlusskappe ohne weitere Manipulation die flexible Membran direkt oder indirekt gegen ein Abheben von der Ventilfläche blockieren. Zur Freigabe kann das Verschlussmittel in diesem Fall beispielsweise von einem Benutzer z.B. an einer Sollbruchstelle abgebrochen werden. Bevorzugt ist in diesem Fall die Membran auch elastisch ausgebildet, sodass sie auch ohne Verschlussmittel an der Ventilfläche anliegt. Grundsätzlich sind auch Ausführungsformen gänzlich ohne Verschlussmittel denkbar, wobei in diesem Fall aber bei der Lagerung der Vorrichtung unter anderem das Risiko einer ungewollten Fluidabgabe besteht.

Der erfindungsgemässe Gedanke erstreckt sich auch auf ein Verschlussstück für eine Vorrichtung für die Aufnahme und Abgabe eines Fluids, insbesondere für eine erfindungsgemässe Vorrichtung wie vorliegend beschrieben. Das Verschlussstück gemäss der Erfindung ist durch die Merkmale des Anspruchs 13 definiert. Gemäss diesem Aspekt der Erfindung umfasst ein Verschlussstück für einen Behälter einen Anschlussbereich zum Anschluss eines Behälters, insbesondere einer Behälterwand, wobei im Anschlussbereich eine Entnahmeöffnung derart angeordnet ist, dass sie mit einem Innenraum des Behälters kommuniziert, wenn der Behälter an das Verschlussstück angeschlossen ist. Weiter umfasst das Verschlussstück einen Abgabebereich, in welchem zur Abgabe des Fluids eine Abgabeöffnung ausgebildet ist, sowie einen Abgabekanal, welcher die Abgabeöffnung fluidmässig mit der Entnahmeöffnung verbindet, wobei ein Abgabeabschnitt des Abgabekanals mit der Abgabeöffnung kommuniziert und ein Entnahmeabschnitt des Abgabekanals mit der Entnahmeöffnung kommuniziert. Der Abgabekanal umfasst dabei eine Ventilvorrichtung, welche in einer Schliessstellung den Abgabekanal fluiddicht verschliesst und einen Durchtritt des Fluids durch den Abgabekanal ermöglicht, wenn in einem Fluid im Abgabekanal ein hinreichend grosser Überdruck besteht. Dieser Aspekt der Erfindung zeichnet sich dadurch aus, dass die Ventilvorrichtung zwei nebeneinander auf einer Ventilfläche angeordnete Ventilöffnungen aufweist, welche insbesondere auf einer gemeinsamen Ventilfläche angeordnet sind, von welchen eine erste Ventilöffnung über den Abgabeabschnitt des Abgabekanals mit der Abgabeöffnung kommuniziert und eine zweite Ventilöffnung über den Entnahmeabschnitt des Abgabekanals mit der Entnahmeöffnung kommuniziert, und eine durchgehende flexible Membran vorhanden ist, welche in der Schliessstellung der Ventilvorrichtung abhebbar an der Ventilfläche anliegt und so die Ventilöffnungen fluiddicht gegeneinander abschliesst.

Der erfindungsgemässe Gedanke betrifft auch losgelöst von der erfindungsgemässen Vorrichtung oder dem Verschlussstück eine Ventilvorrichtung alleine, welche für ein Verschlussstück wie oben beschrieben geeignet ist. Die Ventilvorrichtung umfasst dabei zwei Ventilöffnungen, welche jeweils mit einem Abschnitt eines Fluidkanals kommunizieren und nebeneinander auf einer Ventilfläche angeordnet sind, insbesondere auf einer gemeinsamen Ventilfläche, wobei eine durchgehende flexible Membran vorhanden ist, welche in einer Schliessstellung der Ventilvorrichtung abhebbar an der Ventilfläche anliegt und die Ventilöffnungen fluiddicht gegeneinander abschliesst.

Abwandlungen und bevorzugte Ausführungsformen des Verschlussstücks sowie der Ventilvorrichtung alleine ergeben sich unmittelbar aus der obigen Beschreibung im Zusammenhang mit der erfindungsgemässen Vorrichtung zur Aufnahme und zur Abgabe eines Fluids.

Weiter betrifft die Erfindung auch eine Abgabevorrichtung für eine Vorrichtung für die Aufnahme und Abgabe eines Fluids, insbesondere für eine vorliegend beschriebene erfindungsgemässe Vorrichtung, wobei das Fluid bevorzugt eine Flüssigkeit ist, insbesondere umfassend ein Medikament. Die Abgabevorrichtung weist einen Aufnahmeraum auf, in welchem die Vorrichtung für die Aufnahme und Abgabe eines Fluids angeordnet sein kann, und umfasst eine Betätigungsvorrichtung, mit welcher im Innenraum eines Behälters der Vorrichtung für die Aufnahme und Abgabe eines Fluids ein Überdruck zur Abgabe des Fluids erzeugt werden kann. Bevorzugt ist der Behälter zumindest teilweise deformierbar ausgestaltet, womit auf einfache Weise durch Deformation eines dafür vorgesehenen Bereichs des im Aufnahmeraum angeordneten Behälters der Überdruck erzeugt werden kann. Es versteht sich, dass die Abgabevorrichtung sowohl alleine als auch mit bereits darin angeordneter Vorrichtung zur Aufnahme und Abgabe eines Fluids hergestellt und vertrieben werden kann.

Bevorzugt ist die Betätigungsvorrichtung derart ausgebildet, dass der Behälter bei Betätigung gestaucht oder vorzugsweise gequetscht wird. Eine Quetschung kann dabei z.B. durch einen in Betätigungsrichtung längs geschlitzten Stössel der Betätigungsvorrichtung erreicht werden, welcher in Längsrichtung in den Aufnahmeraum eingeschoben werden kann. Der Stössel wird mit zwei den Schlitz begrenzenden Hälften von einem dem Verschlussstück gegenüberliegenden Längsende des Beutels her mit dem Schlitz über den Beutel geschoben. Im Schlitz werden die Behälterwände des Beutels durch den Vorschub des Stössels zusammengepresst, womit der für eine Fluidabgabe erforderliche Überdruck im Innenraum erzeugt wird. Der Stössel kann dabei direkt durch einen Benutzer betätigt werden oder aber auch z.B. über eine Spindel oder Zahnstange motorisch oder indirekt von einem Benutzer im Aufnahmeraum verschoben werden.

In einer weiteren bevorzugten Ausführungsform weist die Betätigungsvorrichtung der Abgabevorrichtung einen Stössel auf, welcher von der Seite her, d.h. im Wesentlichen quer zur Längsrichtung, in den Aufnahmeraum eingebracht, z.B. eingeschwenkt oder eingeschoben werden kann. In diesem Fall wird der erforderliche Überdruck im Behälter durch Zusammenquetschen des Behälters zwischen Stössel und einem Bereich der Innenwand des Aufnahmeraums erreicht.

In beiden Fällen ist der Stössel derart ausgebildet und dem Aufnahmeraum angepasst, dass ein möglichst vollständiges Ausbringen des Fluids aus dem Behälter ermöglicht wird. Es versteht sich, dass die oben beispielhaft für einen Beutel beschriebenen Betätigungsvorrichtungen auch für eine Tube oder andere kollabierbare Behälter geeignet sind.

Aus der nachfolgenden Detailbeschreibung und der Gesamtheit der Patentansprüche ergeben sich weitere vorteilhafte Ausführungsformen und Merkmalskombinationen der Erfindung.

### Beschreibung der Zeichnungen

Die zur Erläuterung der Ausführungsbeispiele verwendeten Zeichnungen zeigen schematisch:
- Fig. 1a: eine äussere Schrägansicht auf ein Verschlussstück für eine erfindungsgemässe Vorrichtung;
- Fig. 1b: eine seitliche Aussenansicht auf das Verschlussstück gemäss Fig. 1a;
- Fig. 1c: eine Aussenansicht auf eine Grundfläche des Verschlussstücks gemäss Fig. 1a;
- Fig. 1d: eine Aussenansicht auf eine Stirnseite des Verschlussstücks gemäss Fig. 1a;
- Fig. 1e: eine Schnittansicht des Verschlussstücks gemäss Fig. 1a in der Ebene A;
- Fig. 1f: eine Schnittansicht des Verschlussstücks gemäss Fig. 1a in der Ebene B;
- Fig. 2a: eine äussere Schrägansicht auf eine Anschlusskappe für das Verschlussstück gemäss Fig. 1a;
- Fig. 2b: eine Schnittansicht der Anschlusskappe gemäss Fig. 2a in der Ebene C;
- Fig. 2c: einen Ausschnitt der Schnittansicht der Anschlusskappe gemäss Fig. 2b;
- Fig. 3a: eine Schnittansicht in Längsrichtung durch eine Abgabevorrichtung mit darin angeordneter erfindungsgemässer Vorrichtung;
- Fig. 3b: eine Ansicht der Abgabevorrichtung gemäss Fig. 3a mit aufgesetzter Verschlusskappe;
- Fig. 3c: eine Ansicht der Abgabevorrichtung gemäss Fig. 3a, ohne Verschlusskappe mit Verschlussmittel in der Freigabestellung;
- Fig. 3d: eine Ansicht der Abgabevorrichtung gemäss Fig. 3a, nach Betätigung und einer teilweisen Abgabe eines Fluids aus der erfindungsgemässen Vorrichtung;
- Fig. 4a: eine Schnittansicht in Längsrichtung durch eine weitere Ausführungsform eines Verschlussstücks für eine erfindungsgemässe Vorrichtung zur Aufnahme und Abgabe eines Fluids;
- Fig. 4b: eine Schnittansicht in Längsrichtung durch das Verschlussstück gemäss Fig. 4a mit aufgesetzter Verschlusskappe;
- Fig. 4c: eine äussere Schrägansicht des Verschlussstücks gemäss Fig. 4a mit aufgesetzter Verschlusskappe;
- Fig. 5: eine Schnittansicht in Längsrichtung durch eine weiteren Ausführungsform einer Verschlusskappe mit rohrförmigem Kopplungsmittel;
- Fig. 6a: eine Ansicht einer weiteren Ausführungsform einer Abgabevorrichtung mit darin angeordneter erfindungsgemässer Vorrichtung zur Aufnahme und Abgabe eines Fluids;
- Fig. 6b: eine Ansicht der Abgabevorrichtung gemäss Fig. 6a mit einer Anschlusskappe mit Applikationsmittel;

Grundsätzlich sind in den Figuren gleiche Teile mit gleichen Bezugszeichen versehen.

### Wege zur Ausführung der Erfindung

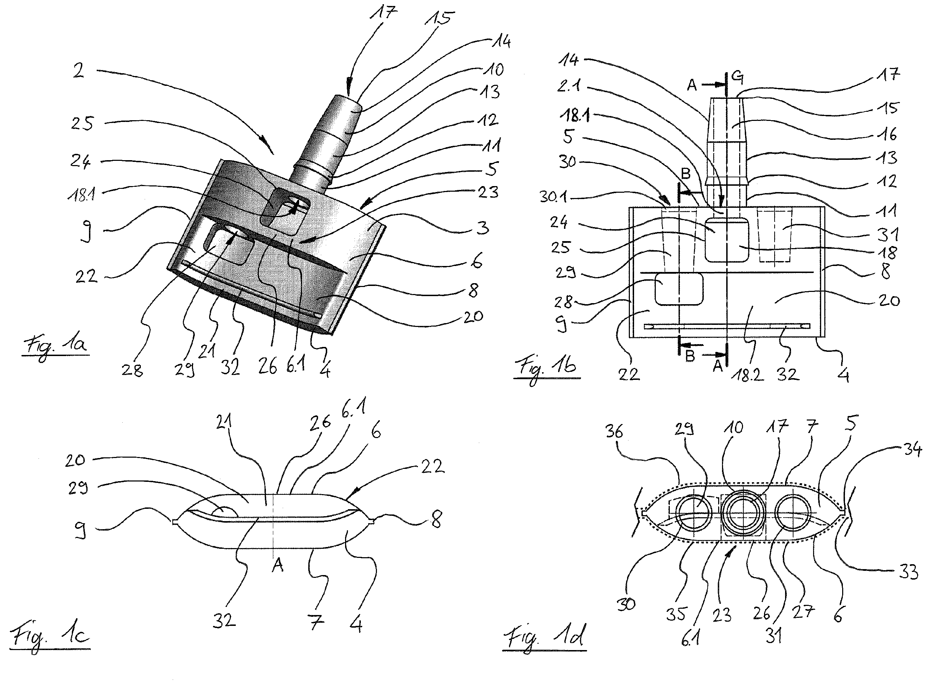

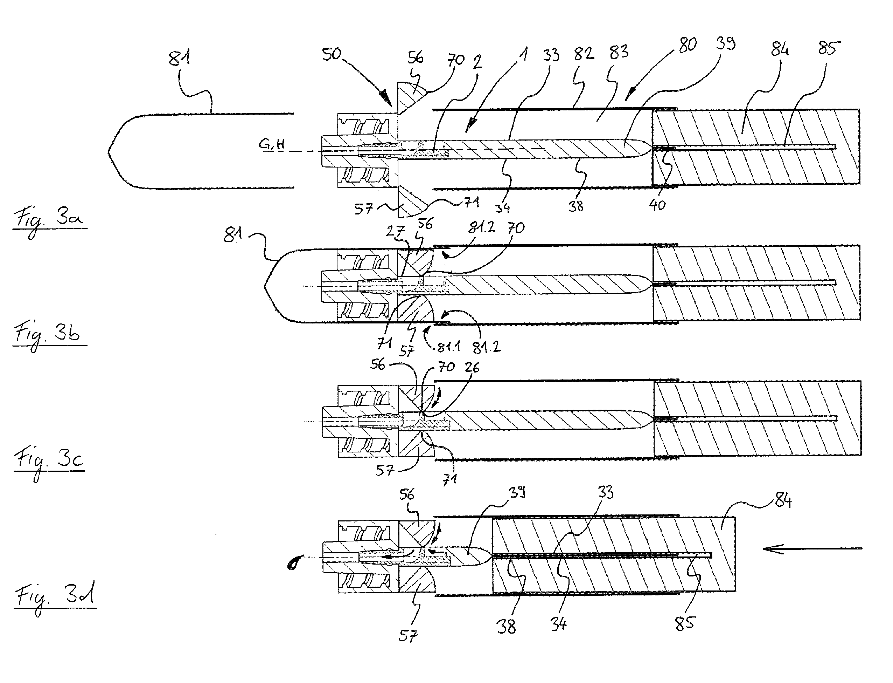

Figur 1a zeigt eine äussere Schrägansicht auf ein Verschlussstück 2 für eine erfindungsgemässe Vorrichtung 1 (siehe Fig. 3a-3d). Das Verschlussstück 2 umfasst einen zylindrischen Grundkörper 3 mit einer linsenförmigen Grundfläche 4 und einer parallel zur Grundfläche 4 ausgerichteten, ebenfalls linsenförmigen Stirnfläche 5. Im Folgenden wird eine Richtung zu einem grundseitigen Längsende des zylindrischen Grundkörpers 4 bzw. über das grundseitige Längsende hinaus mit "nach unten" bzw. "unten" bezeichnet. Entsprechend ist eine Richtung zu einem stirnseitigen Ende hin bzw. über dieses hinaus mit "nach oben" bzw. "oben" bezeichnet.

Eine Mantelfläche des zylindrischen Grundkörpers 3 umfasst zwei Anschlussflächen 6 und 7, welche paarweise in jeweils einer in Richtung einer Zylinderachse G ausgerichteten Kante 8 und 9 zusammenlaufen. Die Kanten 8 und 9 sind dabei bezüglich der Zylinderachse G gegenüberliegend angeordnet. Vorliegend sind die Kanten 8 und 9 als Längsstege ausgebildet, welche die Anschlussflächen 6 und 7 begrenzen.

An der Stirnseite 5 ist ein Kopplungselement 10 am Grundkörper 3 ausgebildet, welches sich längs der Zylinderachse G von der Stirnseite 5 weg erstreckt. In einem grundkörpernahen Abschnitt 11 ist das Kopplungselement 10 kreiszylindrisch ausgebildet. An den kreiszylindrischen Abschnitt 11 schliesst ein als ringförmiger Wulst ausgebildetes Rastmittel 12 an, welches radial bezüglich der Zylinderachse G nach aussen auskragt. An den Wulst 12 schliesst ein weiterer kreiszylindrischer Abschnitt 13 an, welcher sich in einen konischen Abschnitt 14 zu einem grundkörperfernen Längsende 15 des Kopplungselements 10 hin fortsetzt. Ein Durchmesser bezüglich der Zylinderachse G des Kopplungselements 10 ist an jeder Stelle kleiner bemessen, als ein kleinster Durchmesser des Grundkörpers 3.

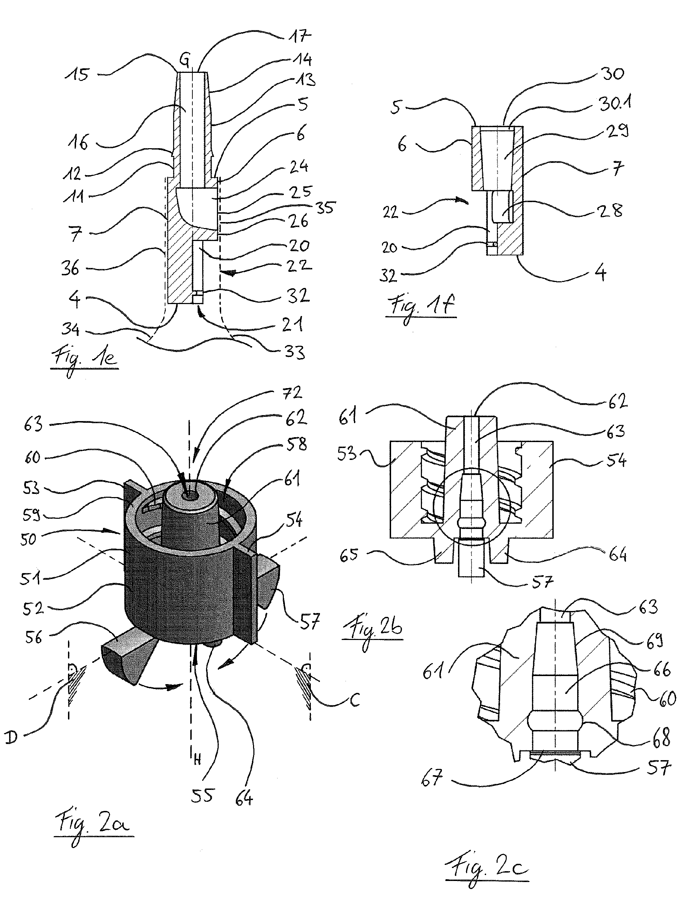

Der Grundkörper 3 weist in einem unteren Bereich eine Aussparung 20 auf. Die Aussparung 20 ist an der Grundseite 4 offen und bildet so an der Grundseite 4 eine Entnahmeöffnung 21 aus, durch welche ein Fluid von der Grundseite 4 her in die Aussparung 20 eintreten kann. Mantelseitig ist die Aussparung 20 an der Anschlussfläche 6 ebenfalls offen ausgebildet, sodass sich eine erste Ventilöffnung 22 einer Ventilvorrichtung 23 ergibt. In Längsrichtung erstreckt sich die Aussparung 20 etwa über die Hälfte der Länge des Grundkörpers 3 - ist mit anderen Worten somit in einer unteren Hälfte angeordnet. In Querrichtung erstreckt sich die Aussparung 20 beinahe bis an die Kanten 8 und 9. Zwischen Kanten 8 und 9 und Aussparung 20 setzt sich jeweils ein schmaler Bereich der Anschlussfläche 6 in Längsrichtung G nach unten zur Grundseite 4 hin fort. In diesen Bereichen kann ein Anschlussabschnitt einer Behälterwand (in Fig. 1 nicht dargestellt) bis zur Grundfläche 4 hin an der Anschlussfläche 6 befestigt werden. Eine in Längsrichtung vordere Kante der von der Aussparung 20 gebildeten Ventilöffnung 22 ist geradlinig ausgebildet und weitgehend senkrecht zur Längsrichtung G ausgerichtet.

In einer oberen Hälfte der Anschlussfläche 6 ist quer zur Längsrichtung G etwa mittig angeordnet eine weitere Aussparung 24 ausgebildet. Die zweite Aussparung 24 ist mantelseitig an der Anschlussfläche 6 offen und bildet eine zweite Ventilöffnung 25 der Ventilvorrichtung 23. In Querrichtung erstreckt sich die Aussparung 24 in etwa über ein Fünftel der Querabmessung der Anschlussfläche 6, wobei die Ventilöffnung 25 weitgehend quadratisch ausgebildet ist. Eine der Aussparung 20 zugewandte Kante der Aussparung 24 ist geradlinig ausgebildet und ist weitgehend parallel zur Vorderkante der Aussparung 20 angeordnet. Aussparung 20 und 24 sind in Richtung von G derart voneinander beabstandet, dass die Anschlussfläche 6 einen schmalen Steg 26 zwischen den einander zugewandten Kanten der Ventilöffnungen 22 und 25 bildet. Die Ventilöffnungen 22 und 25 sind somit benachbart auf einer gemeinsamen Ventilfläche 6.1 angeordnet, welche von einem Bereich der Anschlussfläche 6 gebildet ist.

Im Kopplungselement 10 ist in Längsrichtung G ein Fluidkanal 16 ausgebildet, welcher sich durch das Kopplungselement 10 erstreckt und am Längsende 15 an einer Abgabeöffnung 17 mündet und am gegenüberliegenden Längsende an eine Abgabeöffnung 2.1 des Verschlussstücks 2 anschliesst. Im Grundkörper 3 erstreckt sich ein Abgabekanal 18 von der Abgabeöffnung 2.1 hin zur Entnahmeöffnung 21. Der Abgabekanal 18 umfasst dabei einen Abgabeabschnitt 18.1, welcher die Abgabeöffnung 2.1 fluidmässig mit der Ventilöffnung 26 verbindet, sowie einen Entnahmeabschnitt 18.2, welcher die Ventilöffnung 22 mit der Entnahmeöffnung 21 verbindet. Vorliegend ist der Entnahmeabschnitt 18.2 von der Aussparung 20 gebildet.

In funktionsbereitem Zustand ist die Anschlussfläche 6 von einer flexiblen Membran 27 überdeckt (angedeutet in Fig. 1d), welche zur besseren Übersichtlichkeit in den Fig. 1a-1c nicht dargestellt ist. Insbesondere überdeckt die Membran 27 die Ventilöffnungen 22 und 25 und ist an der Anschlussfläche 6 derart befestigt, dass sie im Bereich des Stegs 26 von der Anschlussfläche 6 abhebbar ist. Liegt die Membran fluiddicht am Steg 26 an, kann ein Fluid nicht von der Aussparung 20 zur Aussparung 24 gelangen. Ist die Membran 27 hingegen vom Steg 26 abgehoben, ist zwischen Steg 26 und Membran 27 ein Fluidkanal gebildet, welcher mit den Ventilöffnungen 22 und 25 kommuniziert und somit einen Fluidurchgang zwischen den Aussparungen 20 und 24 ermöglicht. Die Membran 27 ist dabei bevorzugt in einem Bereich vom Steg 26 abhebbar, welcher in etwa eine Querabmessung aufweist, welcher der Querabmessung der Aussparung 24 entspricht und bis an die einander zugewandten Kanten der Ventilöffnungen 22 und 25 heranreicht. Zumindest in den angrenzenden Bereichen der Anschlussfläche 6, insbesondere bis an die Ventilöffnungen 22 und 25 heran, ist die flexible Membran 27 fest mit der Anschlussfläche 6 verbunden.

Innerhalb der Aussparung 20 ist eine weitere Aussparung 28 ausgebildet, welche bezüglich der Zylinderachse G seitlich versetzt angeordnet ist und bis an eine vordere Begrenzung der Aussparung 20 heranreicht. Die Aussparung 28 ist zur Aussparung 20 hin offen, sodass die Aussparungen 20 und 28 miteinander kommunizieren.

Parallel zur Längsachse G ist ein weiterer Fluidkanal 29 am Grundkörper 3 ausgebildet, welcher an der Stirnseite 5 an einer Anschlussöffnung 30 offen ist und sich im Grundkörper 3 bis zur Aussparung 28 fortsetzt und dort mündet. Der Fluidkanal 29 ist zur Aussparung 28 hin konisch zusammenlaufend ausgebildet (siehe Fig. 1b), sodass ein entsprechender Konus einer Fluidquelle zum Befüllen eines mit dem Verschlussstück 2 versehenen Behälters angeschlossen werden kann. In der dargestellten Ausführungsform gelangt ein über den Fluidkanal 29 zugeführtes Fluid über die Aussparung 28 in die Aussparung 20 und von dort über die Öffnung 21 in einen an das Verschlussstück 2 angeschlossenen Behälter. Die Entnahmeöffnung 21 bildet somit gleichzeitig eine Füllöffnung, über welche der Behälter über die Anschlussöffnung 30 befüllt werden kann. Das System Fluidkanal 29, Aussparung 28 und Aussparung 20 wirkt somit als Füllkanal des Verschlussstücks 2.

Figur 1b zeigt eine seitliche Draufsicht auf das Verschlussstück 2 gemäss Fig. 1a in Richtung auf die Anschlussfläche 6, wobei verdeckte Strukturen gestrichelt angedeutet sind.

Wie sich aus Fig. 1b ergibt, ist bezüglich der Zylinderachse G der Anschlussöffnung 30 gegenüberliegend ein entsprechendes Sackloch 31 an der Stirnseite 5 ausgebildet. Das Sackloch 31 ist dabei weitgehend analog zum Fluidkanal 29 ausgebildet, endet aber in Längsrichtung in einem Abstand einer vorderen Begrenzung der Aussparung 20. Das Sackloch 31 dient dabei zur Aufnahme eines nicht benötigten Verschlusszapfens einer Anschlusskappe, welcher zum Verschliessen der Anschlussöffnung 30 vorgesehen ist (siehe unten, z.B. Fig. 2a-2c). Figur 1b zeigt zwei Schnittflächen A und B, von denen die Ebene A die Längsachse G umfasst und senkrecht auf der Anschlussfläche 6 steht und die Ebene B parallel zur Ebene A im Bereich des Fluidkanals 29 angeordnet ist. Die entsprechenden Schnittabbildungen sind in den Fig. 1e bzw. 1f dargestellt.

Figur 1c zeigt eine Draufsicht auf die Grundseite 4 des Verschlussstücks 2. Nahe der Grundseite 4 ist innerhalb der Aussparung 20 eine Rippe 32 angeordnet. Die Rippe 32 dient dabei als Abklemmrippe 32, auf welche die flexible Membran 27 nach der Befüllung des Behälters 38 mechanisch von aussen angepresst werden kann, bis der Fluidkanal 29 mit einem Verschlusszapfen 64 bzw. 65 der Anschlusskappe 50 verschlossen ist.

Figur 1d zeigt eine Draufsicht auf die Stirnseite 5 des Verschlussstücks 2, wobei verdeckte Strukturen gestrichelt angedeutet sind. Auf den Anschlussflächen 6 und 7 sind Behälterwände 33 und 34 angedeutet, welche mit Anschlussabschnitten 35 und 36 auf den Anschlussflächen 6 bzw. 7 befestigt sind. Der Anschlussabschnitt 35 ist im Bereich der Ventilöffnungen 22 und 25 sowie des Stegs 26 flexibel ausgestaltet und bildet in diesen Bereichen die oben genannte flexible Membran 27 der Ventilvorrichtung 23. Insbesondere ist der Anschlussabschnitt 35 im Bereich des Stegs 26 von der Anschlussfläche 6 abhebbar.

Figur 1e zeigt einen Längsschnitt durch das Verschlussstück 2 in der Ebene A. Der Fluidkanal 16 des Kopplungselements 10 erstreckt sich von dem Längsende 15 bis an die Aussparung 24 und ist über seinen gesamten Querschnitt am Längsende 15 (Abgabeöffnung 17) sowie an der Aussparung 24 offen. Die Aussparung 24 erstreckt sich hierzu entsprechend tief in den Grundkörper 3 hinein. Mit "Tiefe" ist hierbei eine Abmessung in Richtung quer zur Zylinderachse G parallel zur Ebene A, d.h. weitgehend senkrecht zu den Anschlussflächen 6 und 7, bezeichnet. Wie aus Fig. 1e hervorgeht, erstreckt sich die Aussparung 20 im Bereich der Ebene A in Richtung quer zur Zylinderachse G bis zu einer Tiefe in den Grundkörper 3, welche weitgehend der Lage der Längsachse G entspricht. Die Tiefe der Aussparung 20 kann jeweils an die spezifischen Erfordernisse angepasst sein. Beispielsweise kann durch Reduktion dieser Tiefe das Totvolumen der Aussparung 20 reduziert werden.

Figur 1f zeigt einen Längsschnitt durch das Verschlussstück 2 in der Ebene B. Der Fluidkanal 29 verjüngt sich ausgehend von der Anschlussöffnung 30 an der Stirnseite 5 konisch zur Aussparung 28 hin. An der Stirnseite 5 ist der Fluidkanal 29 über seinen gesamten Querschnitt an der Anschlussöffnung 30 offen. An der Anschlussöffnung 30 weist der Fluidkanal 29 einen schmalen zylindrisch ausgebildeten Abschnitt 30.1 auf.

Im Bereich der Ebene B erstreckt sich die Aussparung 20 in Richtung quer zur Zylinderachse G weitgehend bis zur der Lage der Achse G. Die Aussparung 28 ist als Vertiefung in der Aussparung 20 derart ausgebildet und bemessen, dass der Fluidkanal 29 über seinen gesamten Querschnitt offen ist. Vordere Begrenzungswände der Aussparungen 20 und 28 fallen dabei zusammen, wobei die aussparungsseitige Öffnung des Fluidkanals 29 an der vorderen Begrenzungswand ausgebildet ist. Die Aussparungen 24 und 28 weisen weitgehend dieselbe Tiefe auf.

Figur 2a zeigt eine äussere Schrägansicht auf eine Anschlusskappe 50 für eine erfindungsgemässe Vorrichtung 1, insbesondere zum Aufsetzen auf das Verschlussstück 2. Die Anschlusskappe 50 weist einen weitgehend kreiszylindrisch ausgebildeten Grundkörper 51 mit einer Zylinderachse H auf. Bezüglich der Zylinderachse H sind auf einer Mantelfläche 52 des Grundkörpers 51 gegenüberliegend zwei in Richtung der Achse H und in einer Ebene C angeordnete Stege 53 und 54 ausgebildet, welche sich über die gesamte Länge des Grundkörpers 51 erstrecken. Die Stege 53 und 54 geben beim Einlegen der Vorrichtung 1 in eine Abgabevorrichtung eine Ausrichtung der Anschlusskappe 50 vor.

Eine Grundfläche 55 des Grundkörpers 51 ist zum Aufsetzen auf das Verschlussstück 2 vorgesehen und ausgebildet (siehe Fig. 3a-3c). An einem Übergang von der Grundfläche 55 auf die Mantelfläche 52 weist die Verschlusskappe 50 zwei Verschlussmittel 56 und 57 auf, die bezüglich der Zylinderachse H gegenüberliegend und in einer senkrecht zur Ebene C angeordneten Ebene D angeordnet sind. Die Verschlussmittel 56 und 57 sind kreiszylindersegmentartig ausgebildet und mit einer Spitze am Grundkörper 51 der Anschlusskappe 50 derart gelenkig angeformt, dass sie zur Längsachse hin geschwenkt werden können. In der Darstellung der Fig. 2a sind die Verschlussmittel 56 und 57 in einer Bereitschaftsstellung und kragen bezüglich der Zylinderachse H seitlich aus. Die Verschlussmittel 56 und 57 dienen unter anderem zur Blockierung der Ventilvorrichtung 23 in einem Lager- bzw. Transportzustand der Vorrichtung 1 und sind in den Fig. 3a-3d näher beschrieben.

Der Grundkörper 51 weist einen Hohlraum 58 auf, welcher an einer der Grundfläche 55 gegenüberliegenden Stirnseite 59 nach vorne offen ist. An einer Innenwand des Hohlraums 58 ist ein Gewinde 60 ausgebildet. Im Hohlraum 58 ist ein in Längsrichtung H ausgerichteter Konus 61 ausgebildet, welcher an einem vorderen Längsende eine Abgabeöffnung 62 aufweist, die mit einem im Konus 61 ausgebildeten Fluidkanal 63 kommuniziert.

Hohlraum 58 zusammen mit dem Gewinde 60 sowie dem Konus 61 bilden Teile eines bekannten Luer-Lock Anschlusses als Kopplungsmittel 72 der Anschlusskappe 50. Die Dimensionierungen der Teile sind gemäss den bekannten, normierten Standards gewählt, sodass die Anschlusskappe 50 an entsprechend normierte, komplementäre Anschlüsse gekoppelt werden kann.

An der Grundfläche 55 sind bezüglich der Zylinderachse H gegenüberliegend zwei Verschlusszapfen 64 und 65 ausgebildet, welche sich von der Grundfläche 55 weg in Richtung von H erstrecken (siehe z.B. Fig. 2b). Die Verschlusszapfen 64 und 65 sind als von der Grundfläche 55 weg konisch zusammenlaufende Kegelstümpfe ausgebildet, welche derart bemessen sind, dass sie jeweils in den Fluidkanal 29 oder in das Sackloch 31 an der Stirnseite 5 des Verschlussstücks 2 eingebracht werden können. Fluidkanal 29 bzw. Sackloch 31 sind dabei derart im Verhältnis zu den Zapfen 64 und 65 bemessen, dass sich ein Formschluss nach Art einer Konuskopplung ergibt, wenn die Zapfen 64 und 65 vollständig im Fluidkanal 29 bzw. Sackloch 31 angeordnet sind. Die Verschlusszapfen 64 und 65 ermöglichen so einen fluiddichten Abschluss des Fluidkanals 29, wenn die Verschlusskappe 50 auf das Verschlussstück 2 aufgesetzt ist. Indem zwei Zapfen 64 und 65 bezüglich der Zylinderachse H symmetrisch angeordnet sind, kann die Anschlusskappe 50 in zwei Ausrichtungen auf das Verschlussstück 2 aufgesetzt werden, wobei jeweils einer der Anschlusszapfen 64 und 65 im Fluidkanal 29 und der andere im Sackloch 31 angeordnet ist.

Figur 2b zeigt einen Längsschnitt der Anschlusskappe 50 in der Ebene C, während Fig. 2c einen mit einem Kreis in Fig. 2b angedeuteten Ausschnitt vergrössert zeigt. Der im Konus 61 angeordnete Fluidkanal 63 erstreckt sich von der am vorderen Längsende angeordneten Abgabeöffnung 62 hin zur Grundfläche 55 der Anschlusskappe 50. Anschliessend an den Fluidkanal ist ein Aufnahmeraum 66 im Konus 61 ausgebildet, welcher an einer Zugangsöffnung 67 an der Grundfläche 55 in Längsrichtung H offen ist. Der Fluidkanal 63 ist zum Aufnahmeraum 66 hin offen, sodass Aufnahmeraum 66 und Fluidkanal 63 miteinander kommunizieren. Der Aufnahmeraum 66 ist komplementär zum Kopplungselement 10 des Verschlussstücks 2 ausgebildet, sodass dieses durch die Zugangsöffnung 67 in den Aufnahmeraum 66 eingebracht werden kann.

Eine Innenwand des Aufnahmeraums 66 weist den Abschnitten 11 bis 14 des Kopplungselements 10 entsprechende Abschnitte auf, insbesondere eine ringförmige Nut 68, in welcher der ringförmige Wulst 12 des Kopplungselements 10 verrastet ist, wenn dieses im Aufnahmeraum 66 angeordnet ist. Zum Fluidkanal 63 hin weist der Aufnahmeraum 66 einen konisch zusammenlaufenden Abschnitt 69 auf, mit welchem der konische Abschnitt 14 des Kopplungselements 10 nach Art einer Konuskopplung einen fluiddichten Sitz sicherstellt, wenn das Kopplungselement 10 im Aufnahmeraum 66 angeordnet ist.

Figur 3a zeigt einen schematischen Längsquerschnitt durch eine Abgabevorrichtung 80 zur manuellen Abgabe eines Fluids 39 aus der Vorrichtung 1. Die Abgabevorrichtung 80 befindet sich dabei in einem Zustand vor der Erstellung eines Lager- oder Transportzustands. Ein Gehäuse 82 der Abgabevorrichtung 80 weist einen länglichen Aufnahmeraum 83 auf, in welchem die erfindungsgemässe Vorrichtung 1 mit aufgesetzter Anschlusskappe 50 angeordnet ist. Die Anschlusskappe 50 ist dabei derart auf das Verschlussstück 2 aufgesetzt, dass die Achsen G und H koaxial angeordnet sind. Der Aufnahmeraum 83 ist an einem abgabeseitigen Längsende in Längsrichtung nach vorne offen. Die Vorrichtung 1 ist derart im Aufnahmeraum 83 angeordnet, dass die Anschlusskappe 50 wenigstens mit dem als Luer-Lock ausgebildeten Kopplungsmittel aus dem Aufnahmeraum 83 nach vorne übersteht. Die Behälterwände 33 und 34 bilden einen länglichen, beutelartigen Behälter 38, welcher in Längsrichtung der Abgabevorrichtung 80 angeordnet ist. Ein Innenraum des Behälters 38 ist weitgehend vollständig mit dem zur Abgabe vorgesehenen Fluid 39 befüllt. Zur Halterung des Behälters 38 bzw. der Vorrichtung 1 im Aufnahmeraum 83 können seitliche Abschnitte der Behälterwände 33 und 34 am Gehäuse 82 befestigt sein, z.B. von Gehäusehälften eingeklemmt und mit Vorteil mit diesen verschweisst sein (nicht dargestellt).

An einem dem abgabeseitigen Längsende des Grundkörpers 82 gegenüberliegenden Längsende ist ein kolbenartig ausgebildeter Stössel 84 derart angeordnet, dass er von aussen z.B. durch einen Benutzer von hinten her in den Aufnahmeraum 83 eingeschoben werden kann. Der Stössel 84 weist einen Längsspalt 85 auf, welcher zum Aufnahmeraum 83 hin offen ist und sich im Wesentlichen über die gesamte Länge des Stössels 84 erstreckt. In einem vordersten Bereich des Spalts 85 ist ein hinterer Abschlussfalz 40 des Behälters 38 angeordnet. In der Stellung der Fig. 3a ist der Stössel 84 vollständig aus dem Aufnahmeraum 83 ausgefahren.

Die Verschlussmittel 56 und 57 der Anschlusskappe 50 sind in der Bereitschaftsstellung analog zur Darstellung der Fig. 2a vollständig seitlich ausgeklappt. Eine Verschlusskappe 81 ist zum Aufsetzen und Versiegeln des Abgabebereichs der Abgabevorrichtung 80 vorbereitet.

Figur 3b zeigt die Abgabevorrichtung 80 mit aufgesetzter Verschlusskappe 81 in einem Transport- oder Lagerzustand. Die Verschlusskappe 81 ist dabei von vorne her über die Anschlusskappe 50 gestülpt und am Gehäuse 82 der Abgabevorrichtung 80 z.B. über eine Sollbruchstelle 81.1 lösbar befestigt, z.B. angeschweisst. Die Verschlusskappe 81 überspannt dabei die Anschlusskappe 50 sowie den Abgabebereich vollständig und versiegelt diesen gegen eine Kontamination mit Schmutz oder Keimen, insbesondere steril. Vor der Benutzung muss die Verschlusskappe 81 abgenommen werden, wobei die z.B. als Schmutzsiegel ausgebildete Sollbruchstelle erbrochen werden muss.

Die Verschlusskappe 81 ist derart bemessen, dass beim Aufsetzen die Verschlussmittel 56 und 57 der Anschlusskappe 50 von einer Innenwand 81.2 der Verschlusskappe 81 nach innen, zur Zylinderachse H hin geklappt werden. Insbesondere werden die Verschlussmittel 56 und 57 in eine Aktivstellung geschwenkt. Dabei werden an dem jeweiligen Verschlussmittel 56 und 57 ausgebildete Andruckabschnitte 70 bzw. 71 im Bereich einer Längsposition des Stegs 26 auf das Verschlussstück 2 geschwenkt und gegen dieses gepresst. Das auf der Seite der Ventilöffnungen 22 und 25 angeordnete Verschlussmittel 56 wird dabei mit seinem Andruckabschnitt 70 im Bereich des Stegs 26 zwischen den Ventilöffnungen 22 und 25 auf die flexible Membran 27 gepresst. Die flexible Membran 27 ist auf diese Weise zwischen Andruckabschnitt 70 und Ventilfläche 6.1 eingeklemmt, womit sich das Verschlussmittel 56 in einer Schliessstellung befindet und die Ventilvorrichtung 23 in deren Schliessstellung blockiert. Der erforderliche Anpressdruck des Verschlussmittels 56 wird von der Verschlusskappe 81 aufrechterhalten, welche das Verschlussmittel 56 gegen ein Abheben vom Verschlussstück 2 sperrt.

Das weitere Verschlussmittel 57 wird auf der Seite der Anschlussfläche 7 gegen das Verschlussstück 2 gepresst und stützt so das Verschlussstück 2 an der Verschlusskappe 81 ab. Würde die Anschlusskappe um 180 Grad um die Zylinderachse H gedreht auf das Verschlussstück 2 aufgesetzt, was aufgrund der symmetrischen Ausbildung der Anschlusskappe 50 ohne weiteres möglich wäre, wären die Funktionen der Verschlussmittel 56 und 57 vertauscht.

Figur 3c zeigt die Abgabevorrichtung 80 in einem abgabebereiten Zustand, in welchem die Verschlusskappe 81 entfernt wurde. Die Verschlussmittel 56 und 57 sind zwar weiterhin in einer Aktivstellung, in welcher sie mit den Anpressabschnitten 70 und 71 auf das Verschlussstück 2 bzw. die Anschlussabschnitte der Behälterwände 33 und 34 abgesenkt sind. Aufgrund der fehlenden Verschlusskappe 81 können die Verschlussmittel 56 und 57 allerdings vom Verschlussstück 2 weggeschwenkt werden und befinden sich daher in einer Freigabestellung. Insbesondere kann in der Freigabestellung somit die flexible Membran 27 im Bereich des Stegs 26 von der Ventilfläche 6.1 abgehoben werden, womit die Ventilvorrichtung 23 in die Offenstellung gebracht werden kann.

Figur 3d zeigt die Abgabevorrichtung 80 in einem teilweise entleerten Zustand, d.h. in welchem das Fluid 39 aus dem Behälter 83 der Vorrichtung 1 teilweise abgegeben ist. Hierzu wurde der Stössel 84 in Richtung nach vorne in den Aufnahmeraum 83 eingeschoben, wodurch sich der Spalt 85 über den Behälter 38 schiebt. Im Spalt 85 werden dabei die Behälterwände 33 und 34 zusammengedrückt, wodurch ein Überdruck im Fluid 39 im Innenraum des Behälters 38 erzeugt wird. Bei Übersteigen eines durch die Ventilvorrichtung 23, und je nach Andruckkraft des Verschlussmittels 56 in der Freigabestellung, vorgegebenen Schwellenwerts gelangt die Ventilvorrichtung 23 bei ausreichendem Überdruck durch Abheben der Membran 27 in die Offenstellung, sodass das Fluid 39 durch die Entnahmeöffnung 21 in die Aussparung 20 und von dort über die Ventilvorrichtung 23 und den Fluidkanal 16 über die Abgabeöffnung 17 in den Fluidkanal 63 der Anschlusskappe 50 gelangt, wo es zur Abgabeöffnung 62 geführt wird.

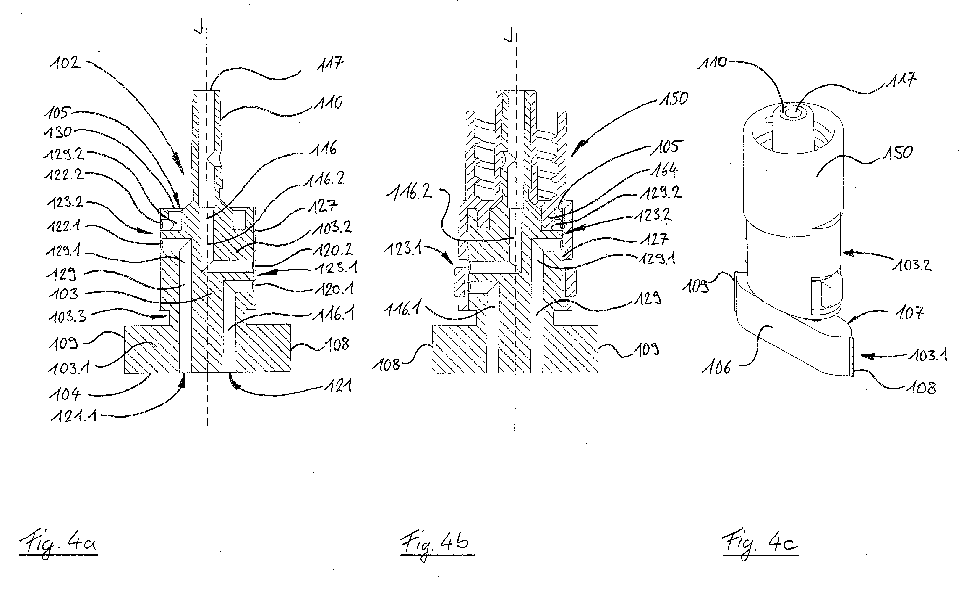

Figuren 4a bis 4c zeigen eine weitere mögliche Ausführungsform eines Verschlussstücks 102 für eine erfindungsgemässe Vorrichtung. Figuren 4a und 4b zeigen Längsschnitte durch das Verschlussstück 102, wobei in Fig. 4b eine Anschlusskappe 150 aufgesetzt ist. Figur 4c zeigt eine äussere Schrägansicht des Verschlussstücks 102 mit aufgesetzter Anschlusskappe 150. Im Folgenden sind die Fig. 4a bis 4c gemeinsam beschrieben.

Das Verschlussstück 102 umfasst einen Grundkörper 103 mit einem zylindrischen Anschlussabschnitt 103.1 mit linsenförmiger Grundfläche 104 und einem in einer Längsrichtung J daran anschliessenden zylindrischen Abschnitt 103.2 mit einem elliptischen Querschnitt. Zwischen den Abschnitten 103.1 und 103.2 ist eine umlaufende Nut 103.3 angeordnet, mit welcher das Verschlussstück 102 in einer entsprechend ausgebildeten Abgabevorrichtung (nicht gezeigt) eingesetzt werden kann.

Der Anschlussabschnitt 103.1 weist zwei Anschlussflächen 106 und 107 auf, welche quer zur Längsrichtung J jeweils in einer gemeinsamen Kante 108 bzw. 109 zusammenlaufen und welche für die Befestigung von Anschlussabschnitten von Behälterwänden (nicht dargestellt) vorgesehen sind.

Auf einer Mantelfläche des zylindrischen Abschnitts 103.2 sind paarweise benachbarte Ventilöffnungen 120.1/2 und 122.1/2 jeweils einer Ventilvorrichtung 123.1 bzw. 123.2 angeordnet. Die Ventilöffnungen 120.1/2 der Ventilvorrichtung 123.1 sind dabei den Ventilöffnungen 122.1/2 der Ventilvorrichtung 123.2 bezüglich der Längsachse J gegenüberliegend angeordnet.

Im Grundkörper 103 ist ein als Abgabekanal ausgebildeter Fluidkanal 116 vorhanden, der einen ersten Abschnitt 116.1 aufweist, welcher eine an der Grundfläche 104 angeordnete Entnahmeöffnung 121 mit der Ventilöffnung 120.1 der Ventilvorrichtung 123.1 fluidmässig verbindet. Ein zweiter Abschnitt 116.2 des Fluidkanals 116 setzt sich von der benachbarten Ventilöffnung 120.2 in Längsrichtung J durch einen an einer Stirnseite 105 des zylindrischen Abschnitts 103.2 angeformtes, als Konus ausgebildetes Kopplungselement 110 bis zu einer Abgabeöffnung 117 fort.

Im Grundkörper 103 ist ein weiterer als Füllkanal ausgebildeter Fluidkanal 129 vorhanden, der einen ersten Abschnitt 129.1 aufweist, welcher eine an der Grundfläche 104 angeordnete Füllöffnung 121.1 mit der Ventilöffnung 122.1 der Ventilvorrichtung 123.2 fluidmässig verbindet. Ein zweiter Abschnitt 129.2 des Fluidkanals 129 setzt sich von der benachbarten Ventilöffnung 122.2 nach vorne bis zu einer an der Stirnseite 105 des zylindrischen Abschnitts 103.2 ausgebildeten Anschlussöffnung 130 fort.

Der zylindrische Abschnitt 103.2 ist von einer manschettenartig ausgebildeten elastischen Membran 127 umfasst, welche insbesondere die Ventilöffnungen 120.1/2 und 122.1/2 überdeckt und fluiddicht an der Mantelfläche des zylindrischen Abschnitts 103.2 anliegt. Die elastische Membran 127 wirkt dabei gleichzeitig als flexible Membran für beide Ventilvorrichtungen 123.1 und 123.2.

Auf den zylindrischen Abschnitt 103.2 ist in der Fig. 4b eine Anschlusskappe 150 aufgesetzt, welche die Membran 127 an die Mantelfläche presst. Im Bereich der Ventilvorrichtung 123.1 ist eine Ausnehmung in der Anschlusskappe 150 ausgebildet, sodass in diesem Bereich die Membran 127 auch bei aufgesetzter Anschlusskappe 150 von der Mantelfläche abhebbar ist. Die Ventilvorrichtung 123.2 ist somit bei aufgesetzter Anschlusskappe 150 in der Schliessstellung blockiert, während die Ventilvorrichtung 123.1 auch bei aufgesetzter Anschlusskappe 150 durch abheben der Membran 127 in die Offenstellung gebracht werden kann. Zum Befüllen ist die Anschlusskappe 150 nicht auf das Verschlussstück 102 aufgesetzt, sodass über den Füllkanal 129 ein angeschlossener Behälter über die Ventilvorrichtung 123.2 befüllt werden kann. Nach dem Befüllen wird die Anschlusskappe 150 aufgesetzt, wodurch der Füllkanal 129 gesperrt ist d.h. die Ventilvorrichtung 123.2 in der Schliessstellung blockiert ist. Dabei gelangt ein Verschlusszapfen 164 der Anschlusskappe 150 in den Abschnitt 129.2 und verschliesst diesen an der Stirnseite 105 fluiddicht.

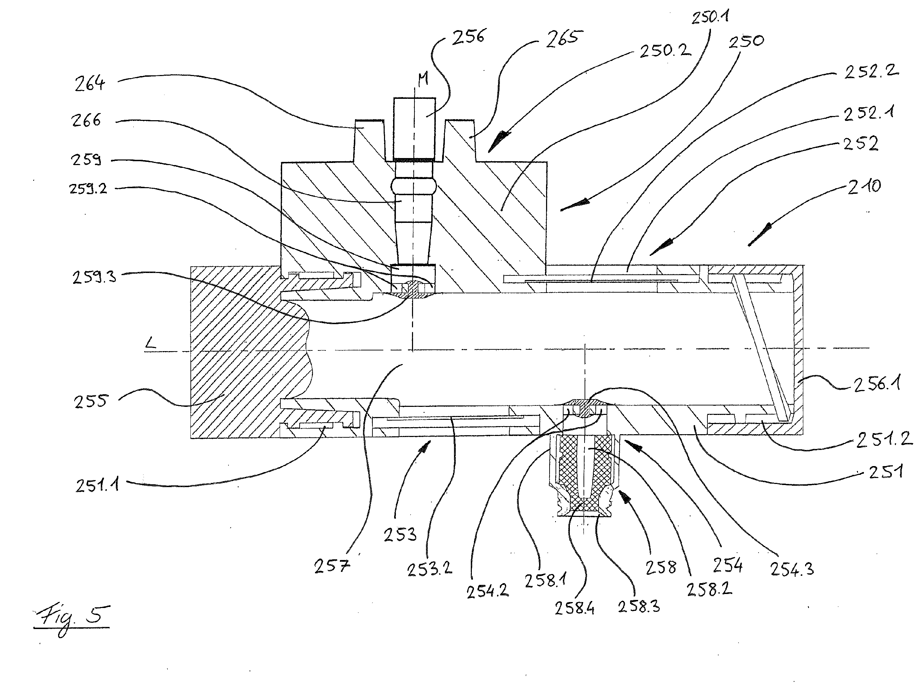

Figur 5 zeigt eine weitere Ausführungsform einer Anschlusskappe 250 für das Verschlussstück 2 mit einem Kopplungsmittel 210 zum Anschluss an ein fluidführendes System, insbesondere zur Anwendung im Bereich von Dialysesystemen. Das Kopplungsmittel 210 ist als länglicher Rohrabschnitt 251 ausgebildet und direkt an einen weitgehend kreiszylindrisch ausgebildeten Grundkörper 250.1 der Anschlusskappe 250 angeformt. Eine Längsrichtung L des Rohrabschnitts 251 ist weitgehend senkrecht zu einer Zylinderachse M des Grundkörpers 250.1 angeordnet. Ein Anschlussbereich 250.2 der Anschlusskappe 250 zum Anschluss einer erfindungsgemässen Vorrichtung 1 umfasst einen Aufnahmeraum 266 für das Kopplungselement 10 des Verschlussstücks 2, ein Verschlussmittel 256 sowie zwei Verschlusszapfen 264 und 265, welche in Form und Funktion weitgehend der z.B. in Fig. 2 bis 3 beschriebenen gleichartigen Elemente entsprechen.

An seinen Endseiten ist der Rohrabschnitt 251 mit jeweils einer Kopplungsstelle 251.1 und 251.2 versehen, welch einen Anschluss des Rohrabschnitts 250 an das fluidführende System (nicht dargestellt) erlaubt. In der Darstellung der Figur 5 ist die Kopplungsstelle 251.1 als Schnappkupplung ausgebildet und mit einer entsprechenden abnehmbaren Verschlusskappe 255 versehen. Die Kopplungsstelle 251.2 ist als Gewindekupplung ausgebildet und weist ebenfalls eine abnehmbare Verschlusskappe 256.1 auf.

Der Aufnahmeraum 266 mündet direkt in eine Zugangsöffnung 259 des Rohrabschnitts 251. Innenseitig an der Rohrwand weist die Zugangsöffnung 259 mehrere Öffnungen 259.2 auf, welche einen Durchtritt von Flüssigkeit aus dem Aufnahmeraum 266 in einen Innenraum 257 des Rohrabschnitts 251 ermöglichen. Die Öffnungen 259.2 sind dabei bevorzugt ringförmig um eine zentral angeordnete Befestigungsstelle eines Schirmventils 259.3 angeordnet. Ein Schirm des Schirmventils 259.3 erstreckt sich in den Innenraum 257 und ist derart ausgebildet, dass er die Öffnungen 259.2 vollständig überdeckt. Der Schirm ist bevorzugt als dünne Dichtlippe ausgebildet, welche sich innenseitig an die Rohrwand schmiegt sich ausgehend von der Befestigungsstelle radial nach aussen verjüngt. An einem Rand des Schirms liegt dieser somit weitgehend stufenlos an der Innenwand des Rohrabschnitts 251 an.

Wird nun ein Fluid von einer angeschlossenen erfindungsgemässen Vorrichtung 1 (in Fig. 5 nicht dargestellt) abgegeben, gelangt das Fluid in die Zugangsöffnung 259. Ist ein Druck im Fluid genügend gross, wird der Schirm des Schirmventils 259.3 von der Rohrinnenwand abgehoben, sodass die Öffnungen 259.2 freigegeben werden und das Fluid in den Innenraum 257 gelangt. Sobald kein Druck mehr im Fluid vorhanden ist, schliesst das Schirmventil 259.3, indem der Schirm sich wieder dichtend über die Öffnungen 259.2 an die Rohrinnenwand legt.

Durch den weitgehend glatten Übergang von der Rohrwand auf den Schirm ergibt sich gesamthaft eine nur geringfügige Beeinträchtigung eines im Innenraum 257 des Rohrabschnitts 251 strömenden Fluids des fluidführenden Systems. Ebenso ist unmittelbar ersichtlich, dass im Innenraum 257 aufgrund der erfindungsgemässen Konstruktion keine Toträume vorhanden sind, in welchen die strömende Flüssigkeit auf unerwünschte Weise verweilen könnte.

Das Kopplungsmittel 210 weist zudem drei Injektionsports 252 bis 254 auf. Ein direkt neben dem Grundkörper 250.1 ausgebildeter Injektionsport 252 umfasst ein in eine Öffnung 252.1 der Rohrwand eingesetztes Septum 252.2. Das Septum 252.2 erlaubt eine selbstschliessende Penetration durch beispielsweise eine Injektionsnadel. Das Septum 252.2 ist derart in der Rohrwand ausgebildet, dass es eine weitgehend direkte Fortsetzung der Rohrwand bildet und so eine strömende Flüssigkeit möglichst nicht behindert bzw. verwirbelt. Das Septum 252.2 ist hierzu direkt an der Rohrwand befestigt wie beispielsweise ultraschall- oder laserverschweisst. Bezüglich der Längsachse L ist der Zugangsöffnung 259 gegenüberliegend in der Rohrwandung ein weiterer Injektionsport 253 ausgebildet. Der Injektionsport 253 ist weitgehend analog zum Injektionsport 252 ausgebildet und umfasst ein Septum 253.2.

Bezüglich der Längsachse M ist dem Injektionsport 252 gegenüberliegend in der Rohrwand ein weiterer Injektionsport 254 ausgebildet. Dieser weist analog der Zugangsöffnung 259 Öffnungen 254.2 sowie ein Schirmventil 254.3 auf. Im Gegensatz zur Zugangsöffnung 259 ist aussenseitig am Rohrabschnitt 251 allerdings ein Kopplungsmittel 258 vorgesehen, welches eine nadellose Ankopplung einer medizinischen Einrichtung zur Fluidabgabe ermöglicht. In der Darstellung der Fig. 5 ist das Kopplungsmittel 258 als so genanntes abtupfbares Ventil ausgebildet ("swabable valve"). Derartige Ventile umfassen einen Kupplungssockel 258.1, welcher vorliegend am Rohrabschnitt angeformt ist. Ein mit den Öffnungen 254.2 in Kommunikation stehender Innenraum 258.2 des Sockels 258.1 weist eine Anschlussöffnung 258.3 mit z.B. einem Innenkonus einer Luer-Kupplung auf. Zur Dichtung gegen Verschmutzung oder Kontamination ist im Innenraum ein Schlitzventil 258.4 angeordnet, welches bei Einbringen eines Aussenkonus zurückgedrängt wird und bei Ausbringen die äussere Öffnung 258.3 wieder verschliesst. Das Schlitzventil 258.4 ist derart in der Öffnung 258.3 angeordnet, dass es von aussen auf einfache Weise abgetupft d.h. gereinigt werden kann. Derartige Kopplungen sind in der US 6,651,956 (Halkey-Roberts Corporation) beschrieben. Figur 5 versteht sich als schematische Ansicht, weshalb ein Grössenverhältnis z.B. zwischen der Anschlusskappe 250 zu Kopplungsmittel 258 nicht dem tatsächlichen Verhältnis entsprechen muss.

Es versteht sich, dass der Rohrabschnitt 251 beispielsweise auch direkt in ein Rohr oder einen Schlauch integriert sein kann. Ebenso kann der Rohrabschnitt z.B. einseitig fest verschlossen sein und nur an einem Ende zur Ankopplung an ein fluidführendes System vorgesehen sein. Ebenso ist unmittelbar ersichtlich, dass auch andere oder zusätzliche Kopplungsmittel wie z.B. weitere Luer-Locks und auch zusätzliche Injektionsports vorgesehen sein können. Im Allgemeinen empfiehlt es sich, dass der Rohrabschnitt zusätzlich zur Möglichkeit zur Ankopplung einer erfindungsgemässen Vorrichtung wenigstens einen weiteren Port aufweist, an welchen eine weitere, gegebenenfalls auch anders geartete Abgabevorrichtung wie beispielsweise eine Spritze angekoppelt werden kann.

Aus Fig. 5 wird unmittelbar klar, dass das Kopplungselement 210 auch direkt an ein Verschlussstück einer erfindungsgemässen Vorrichtung angeformt sein kann. In diesem Fall kann der Grundkörper 3 des Verschlussstücks 2 z.B. mit seiner Stirnseite 5 direkt an den Rohrabschnitt 251 anschliessen wie z.B. einstückig angeformt sein, wobei die Ventilöffnung 25 über einen Fluidkanal direkt mit der Zugangsöffnung 259 kommuniziert.

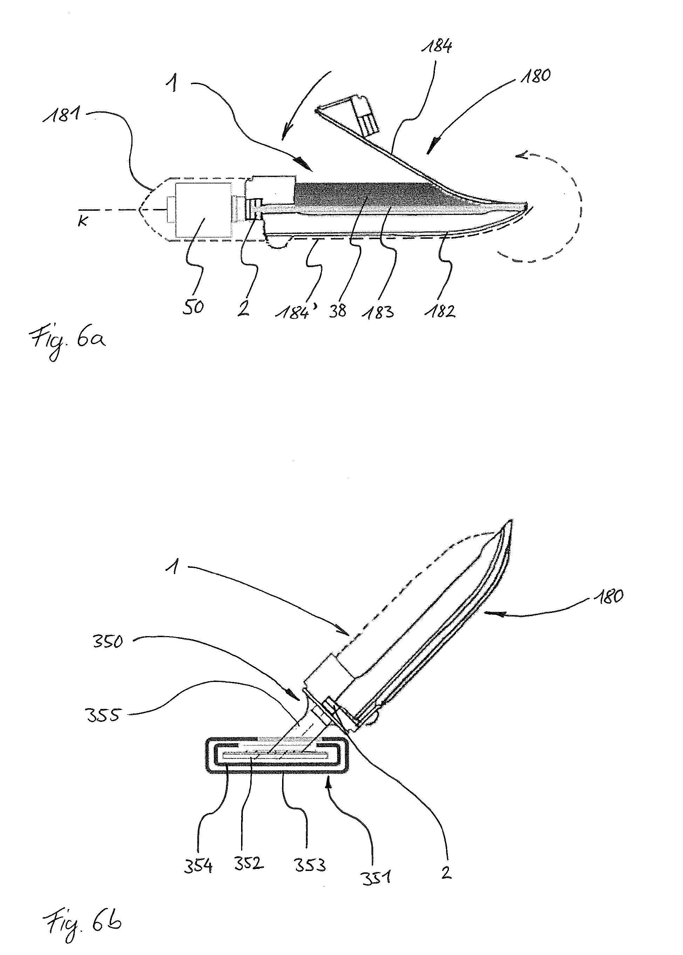

Figur 6a zeigt eine schematische Ansicht einer weiteren Ausführungsform einer Abgabevorrichtung 180 zur manuellen Abgabe eines Fluids 39 aus der Vorrichtung 1. Die Abgabevorrichtung 180 eignet sich besonders beispielsweise zur Abgabe und Applikation eines Desinfektionsmittels. Diese Abgabevorrichtung ist beispielsweise aber auch bei eine Anwendung bei kosmetischen Fluiden oder im Lebensmittelbereich, z.B. für viskose Lebensmittel wie Saucen (Ketchup, Senf etc.) besonders geeignet.

Ein Gehäuse 182 der Abgabevorrichtung 180 weist einen länglichen Aufnahmeraum 183 auf, in welchem die erfindungsgemässe Vorrichtung 1 mit aufgesetzter Anschlusskappe 50 angeordnet ist. Die Anschlusskappe 50 ist in der Abbildung der Fig. 6a ohne Detaillierung nur schematisch angedeutet. Der Aufnahmeraum 183 ist an einem abgabeseitigen Längsende in einer Längsrichtung K nach vorne offen. Die Vorrichtung 1 ist derart im Aufnahmeraum 183 angeordnet, dass das Verschlussstück 2 zusammen mit der Anschlusskappe 50 aus dem Aufnahmeraum 183 nach vorne übersteht.

Das Gehäuse 182 ist quer zur Längsrichtung K weitgehend halbseitig offen ausgebildet, sodass von der Seite her direkt Druck auf den Behälter 38 ausgeübt werden kann. An einem dem abgabeseitigen Längsende des Grundkörpers 182 gegenüberliegenden Längsende, d.h. einem hinteren Längsende, ist ein als Drücker ausgebildeter Hebelarm 184 um eine quer zur Längsachse K angeordnete Schwenkachse schwenkbar angelenkt. Der Drücker 184 ist dabei derart am Gehäuse 182 angeordnet, dass er von der offenen Hälfte her in den Aufnahmeraum 183 einschwenkbar ist (durchgezogener Pfeil). Der Drücker 184 ist entsprechend einer äusseren Form des Gehäuses 182 ausgebildet, sodass er in einem Lager- oder Transportzustand aussenseitig an das Gehäuse 182 geklappt und dort allenfalls verrastet sein kann (Stellung 184', gestrichelt angedeutet). Zur Benutzung wir der Drücker 184 ausgeklappt (gestrichelter Pfeil).

Im Lager oder Transportzustand ist der Abgabebereich der Abgabevorrichtung 180 mit einer Verschlusskappe 181 versehen und versiegelt, wobei die Verschlusskappe 181 analog der Darstellung der Fig. 3b die Verschlussmittel 56 und 57 der Anschlusskappe 50 fixiert.

Die Abgabevorrichtung 180 ist insbesondere auch im Zusammenhang mit einer Anschlusskappe 350 mit einem Applikationsmittel 351 wie beispielsweise einem Schwamm 352 besonders vorteilhaft, wenn das Fluid 39 z.B. ein Desinfektionsmittel zur flächigen Applikation umfasst. Figur 6b zeigt schematisch eine derartige Ausführungsform. Die Anschlusskappe 350 weist hierzu einen Halter 355 für das Applikationsmittel 351 auf, welcher einen innen liegenden Fluidkanal aufweist (nicht dargestellt) der mit der Abgabeöffnung des Verschlussstücks 2 kommuniziert. Der Fluidkanal mündet am Applikationsmittel 351, sodass dieses direkt mit dem Fluid 39 aus der erfindungsgemässen Vorrichtung 1 versorgt werden kann.

Aufgrund der Anordnung des Drückers 184 (in Fig. 6b in einer Transport- oder Lagerstellung) kann die Abgabevorrichtung 180 vom Benutzer auf einfache Weise mit dem Daumen bedient werden, während er mit dem aufgrund der Betätigung mit dem Desinfektionsmittel getränkten Applikationsmittel 351 den zu desinfizierenden Bereich überstreicht. Das Applikationsmittel 351 umfasst dabei den Schwamm 352, welcher vorliegend von zwei Lagen 353 und 354 z.B. eines Vlieses, Netzes oder Mikrofilters umhüllt ist. Die einzelnen Lagen 353 und 354 sind abnehmbar ausgebildet, sodass nach einer Grobreinigung eine äusserste Lage 353 abgenommen werden kann, um mit der weiteren, noch sauberen Lage 354 eine Feinreinigung durchzuführen. Zur endgültigen Desinfektion kann auch die weitere Lage 354 abgenommen werden, sodass die endgültige Desinfektion mit dem noch sauberen Schwamm 352 durchgeführt werden kann.

Die erfindungsgemässe Vorrichtung zur Aufnahme und Abgabe eines Fluids bietet somit vielseitige Anwendungsmöglichkeiten und kann insbesondere in Zusammenhang mit der weitgehend beliebig ausführbaren Anschlusskappe in verschiedensten Gebieten zum Einsatz kommen.

## Patentansprüche

1. Vorrichtung (1) zur Aufnahme oder Abgabe eines Fluids (39), vorzugsweise für ein Fluid umfassend ein Medikament, mit einem Behälter (38), insbesondere einen wenigstens bereichsweise kollabierbaren Behälter, und mit einem Verschlussstück (2), das Verschlussstück (2) umfassend
a) einen Anschlussbereich zum Anschluss des Behälters (38), wobei im Anschlussbereich eine Entnahmeöffnung (21) derart angeordnet ist, dass sie mit einem Innenraum des Behälters (38) kommuniziert, und
b) einen Abgabebereich, in welchem zur Abgabe des Fluids (39) eine Abgabeöffnung (2.1) ausgebildet ist, sowie
c) einen Abgabekanal (18), welcher die Abgabeöffnung (2.1) fluidmässig mit der Entnahmeöffnung (21) verbindet, wobei ein Abgabeabschnitt (18.1) des Abgabekanals (18) mit der Abgabeöffnung (2.1) kommuniziert und ein Entnahmeabschnitt (18.2) des Abgabekanals (18) mit der Entnahmeöffnung (21) kommuniziert,
d) wobei der Abgabekanal (18) eine Ventilvorrichtung (23) umfasst, welche in einer Schliessstellung den Abgabekanal (18) fluiddicht verschliesst und in einer Offenstellung einen Durchtritt des Fluids (39) durch den Abgabekanal (18) ermöglicht, wenn in einem Fluid (39) im Entnahmeabschnitt (18.2) ein hinreichend grosser Überdruck besteht,
**dadurch gekennzeichnet, dass**
die Ventilvorrichtung (23) zwei nebeneinander auf einer Ventilfläche (6.1) angeordnete Ventilöffnungen (22, 25) aufweist, welche insbesondere auf einer gemeinsamen Ventilfläche (6.1) angeordnet sind, von welchen eine erste Ventilöffnung (25) über den Abgabeabschnitt (18.1) des Abgabekanals (18) mit der Abgabeöffnung (2.1) kommuniziert und eine zweite Ventilöffnung (22) über den Entnahmeabschnitt (18.2) des Abgabekanals (18) mit der Entnahmeöffnung (21) kommuniziert, und eine durchgehende flexible Membran (27) vorhanden ist, welche in der Schliessstellung der Ventilvorrichtung (23) bereichsweise abhebbar an der Ventilfläche (6.1) anliegt und so die Ventilöffnungen (22, 25) fluiddicht gegeneinander abschliesst.

2. Vorrichtung (1) nach Anspruch 1, **dadurch gekennzeichnet, dass** eine Behälterwand (33, 34) des Behälters (38) zumindest bereichsweise flexibel, insbesondere als Beutel oder Tube, ausgebildet ist, wobei die Behälterwand (33, 34) vorzugsweise einen Kunststoff umfasst, insbesondere einen beschichteten Kunststoff, vorzugsweise ein Laminat, und das Verschlussstück (2) im Anschlussbereich mit Vorteil wenigstens eine Anschlussfläche (6) aufweist, an welcher ein Anschlussabschnitt (35) der Behälterwand (33) befestigt ist, insbesondere mit der Anschlussfläche (6) verschweisst oder verklebt ist.

3. Vorrichtung (1) nach Anspruch 2, **dadurch gekennzeichnet, dass** der Anschlussabschnitt (35) der Behälterwand (33) flexibel ausgebildet ist und die Ventilfläche (6.1) mit Ventilöffnungen (22, 25) auf der wenigstens einen Anschlussfläche (6) des Verschlussstücks (2) derart angeordnet ist, dass ein Bereich des Anschlussabschnitts (35) der Behälterwand (33) die flexible Membran (27) der Ventilvorrichtung (23) bildet, insbesondere die Ventilöffnungen (22, 25) überdeckt.

4. Vorrichtung nach einem der Ansprüche 2 bis 3, **dadurch gekennzeichnet, dass** das Verschlussstück (2) im Anschlussbereich einen im Wesentlichen zylindrisch ausgebildeten Abschnitt aufweist und die wenigstens eine Anschlussfläche (6) auf einer äusseren Mantelfläche des zylindrischen Abschnitts ausgebildet ist.

5. Vorrichtung (1) nach Anspruch 4, **dadurch gekennzeichnet, dass** die Mantelfläche des zylindrischen Abschnitts neben der wenigstens einen Anschlussfläche (6) eine weitere Anschlussfläche (7) umfasst, wobei die beiden Anschlussflächen (6, 7) paarweise in einer gemeinsamen, insbesondere zur Zylinderachse (G) parallel angeordneten, Kante (8, 9) zusammenlaufen, sodass der zylindrische Abschnitt eine weitgehend linsenförmige Grundfläche (4) aufweist.

6. Vorrichtung (1) nach einem der Ansprüche 1 bis 5, **dadurch gekennzeichnet, dass** das Verschlussstück (2) einen Füllkanal (29) zum Befüllen des Behälters (38) umfasst, welcher eine Anschlussöffnung (30) zum Anschliessen einer Fluidquelle im Abgabebereich und eine Füllöffnung (21) im Anschlussbereich, welche mit dem Innenraum des Behälters (38) kommuniziert, fluidmässig verbindet.

7. Vorrichtung (1) nach einem der Ansprüche 1 bis 6, **dadurch gekennzeichnet, dass** am Verschlussstück (2) im Abgabebereich ein Kopplungselement (10) zum direkten oder indirekten Ankoppeln der Vorrichtung an eine fluidführende Komponente ausgebildet ist, welches Kopplungselement (10) insbesondere einen sich vom Verschlussstück weg verjüngenden Kopplungskonus (14) umfasst und einen Fluidkanal (16) aufweist, welcher an die Abgabeöffnung (2.1) anschliesst.

8. Vorrichtung (1) nach einem der Ansprüche 1 bis 7, **dadurch gekennzeichnet, dass** eine Anschlusskappe (50,250) vorhanden ist, welche auf das Verschlussstück (2) aufsetzbar ist, insbesondere auf das Kopplungselement (10) aufsetzbar und an dieses koppelbar ist, und insbesondere in aufgesetzter Anordnung die Anschlussöffnung (30) im Abgabebereich des Verschlussstücks (2) verschliesst.

9. Vorrichtung (1) nach Anspruch 8, **dadurch gekennzeichnet, dass** die Anschlusskappe (50, 250) ein Kopplungsmittel (72, 210) zur fluidkommunizierenden Ankopplung der Abgabeöffnung (2.1) an ein fluidführendes System aufweist, insbesondere einen Aussenkonus eines Luer-Locks.

10. Vorrichtung (1) nach Anspruch 7 in Verbindung mit einem der Ansprüche 8 oder 9, **dadurch gekennzeichnet, dass** die Anschlusskappe (50, 250) einen weitgehend komplementär zum Kopplungselement (10) des Verschlussstücks (2) ausgebildeten Aufnahmeraum (66, 266) aufweist, in welchem das Kopplungselement (10) bei aufgesetzter Anschlusskappe (50, 250) aufnehmbar ist.

11. Vorrichtung (1) nach einem der Ansprüche 8 bis 10, **dadurch gekennzeichnet, dass** die Anschlusskappe (50, 250) ein Verschlussmittel (56, 256) aufweist, welches derart ausgebildet ist, dass es bei aufgesetzter Anschlusskappe (50, 250) die Ventilvorrichtung (23) in der Schliessstellung fixiert.

12. Vorrichtung nach Anspruch 11, **dadurch gekennzeichnet, dass** das Verschlussmittel (56, 256) derart ausgebildet und angeordnet ist, dass die flexible Membran (27) im Bereich zwischen den Ventilöffnungen (22, 25) durch das Verschlussmittel (56, 256) fest an die Ventilfläche (6.1) pressbar ist.

13. Verschlussstück (2) für eine Vorrichtung zur Aufnahme oder Abgabe eines Fluids (39), vorzugsweise für ein Fluid umfassend ein Medikament, insbesondere für eine Vorrichtung (1) gemäss einem der Ansprüche 1 bis 12, umfassend
a) einen Anschlussbereich zum Anschluss des Behälters (38) der Vorrichtung (1), wobei im Anschlussbereich eine Entnahmeöffnung (21) derart angeordnet ist, dass sie mit einem Innenraum des Behälters (38) kommuniziert, wenn der Behälter (38) an das Verschlussstück (2) angeschlossen ist, und
b) einen Abgabebereich, in welchem zur Abgabe des Fluids (39) eine Abgabeöffnung (2.1) ausgebildet ist,
c) einen Abgabekanal (18), welcher die Abgabeöffnung (2.1) fluidmässig mit der Entnahmeöffnung (2.1) verbindet, wobei ein Abgabeabschnitt (18.1) des Abgabekanals (18) mit der Abgabeöffnung (2.1) kommuniziert und ein Entnahmeabschnitt (18.2) des Abgabekanals (18) mit der Entnahmeöffnung (21) kommuniziert,
**dadurch gekennzeichnet, dass**
das Verschlussstück (2) zwei nebeneinander auf einer Ventilfläche (6.1) angeordnete Ventilöffnungen (22, 25) für eine Ventilvorrichtung (23) aufweist, welche insbesondere auf einer gemeinsamen Ventilfläche (6.1) angeordnet sind, von welchen eine erste Ventilöffnung (25) über den Abgabeabschnitt (18.1) des Abgabekanals (18) mit der Abgabeöffnung (2.1) kommuniziert und eine zweite Ventilöffnung (22) über den Entnahmeabschnitt (18.2) des Abgabekanals (18) mit der Entnahmeöffnung (21) kommuniziert, und dass die Ventilöffnungen (22, 25) derart ausgebildet und angeordnet sind, dass die Ventilöffnungen (22, 25) für eine Schliessstellung der Ventilvorrichtung (23) durch abhebbares Anlegen einer durchgehenden flexible Membran (27) gegeneinander abschliessbar sind.

14. Ventilvorrichtung (23) für eine Vorrichtung zur Aufnahme oder Abgabe eines Fluids, vorzugsweise für ein Fluid umfassend ein Medikament, insbesondere für ein Verschlussstück (2) einer Vorrichtung (1) gemäss einem der Ansprüche 1 bis 12, **dadurch gekennzeichnet, dass** zwei Ventilöffnungen (22, 25) vorhanden sind, welche jeweils mit einem Abschnitt (18.1, 18.2) eines Fluidkanals (18) kommunizieren und nebeneinander auf einer Ventilfläche (6.1) angeordnet sind, insbesondere auf einer gemeinsamen Ventilfläche (6.1), und dass eine durchgehende flexible Membran (27) vorhanden ist, welche in einer Schliessstellung der Ventilvorrichtung (23) abhebbar an der Ventilfläche (6.1) anliegt und die Ventilöffnungen (22, 25) fluiddicht gegeneinander abschliesst.

15. Abgabevorrichtung (80, 180) für eine Vorrichtung zur Aufnahme und Abgabe eines Fluids, insbesondere für eine Vorrichtung (1) gemäss einem der Ansprüche 1 bis 12, welche einen Aufnahmeraum (83, 183) aufweist, in welchem die Vorrichtung (1) für die Aufnahme und Abgabe eines Fluids angeordnet sein kann, und eine Betätigungsvorrichtung (84, 184) umfasst, mit welcher im Innenraum eines Behälters (38) der Vorrichtung (1) für die Aufnahme und Abgabe eines Fluids ein Überdruck zur Abgabe des Fluids erzeugt werden kann.
